# EUROPÄISCHE PATENTSCHRIFT

(11) **EP 0 431 371 B1**
(45) Veröffentlichungstag und Bekanntmachung des Hinweises auf die Patenterteilung: **10.09.1997**
(21) Anmeldenummer: 90121958.4
(22) Anmeldetag: 16.11.1990
(51) Int. Cl.: C07D 207/34, A61K 31/40

(54) **3-Aminopyrrole, Verfahren zu ihrer Herstellung und ihre Verwendung als Antikonvulsiva**
3-Aminopyrroles, process for their preparation and their use as anticonvulsives
3-Aminopyrroles, procédé pour leur préparation et leur utilisation comme anticonvulsifs

(30) Priorität: 17.11.1989 DD 334670
(43) Veröffentlichungstag der Anmeldung: 12.06.1991
(73) Patentinhaber: ARZNEIMITTELWERK DRESDEN GmbH, 01445 Radebeul (DE)
(72) Erfinder: Liebscher, Jürgen, Dr. sc., O-1144 Berlin (DE); Knoll, Alexander, Dr., O-1150 Berlin (DE); Uschmajew, Alexej, Dr., O-1155 Berlin (DE); Rolfs, Andreas, Dipl.-Chem., O-1080 Berlin (DE); Lohmann, Dieter, Dr. sc., O-8122 Radebeul (DE); Faust, Gottfried, Dr., O-8122 Radebeul (DE); Morgenstern, Eveline, Dr., O-1034 Berlin (DE); Scharfenberg, Peter, Dr., W-5600 Wuppertal 2 (DE)
(74) Vertreter: Beetz & Partner Patentanwälte

(56) Entgegenhaltungen:
- EP-A- 0 249 236
- Chemical Abstracts, Band 103, Nr. 15, 14. Oktober 1985, Seite 723, Zusammenfassung Nr. 123423t, Columbus, Ohio, US; A. KNOLL et al.: "Products of formylation of thio amides. 10. Synthesis of esters of substituted 3-amino-2-pyrrolecarboxylic acids from thiacrylamides and glycine esters", & KHIM. GETEROTSIKL. SOEDIN. 1985, (5), 628-30
- Journal of medicinal chemistry, Band 11, 1968, Seiten 1000-1008; R. HOWE et al.: "Beta-adrenergic blocking agents. I. Pronethalol and related N-alkyl and N-aralkyl derivatives of 2-amino-1-(2-naphthyl)ethanol"

## Beschreibung

Die Erfindung betrifft 3-Aminopyrrole, Verfahren zu ihrer Herstellung und ihre Verwendung als Arzneistoffe bzw. Arzneimittel. Die Erfindung ist in der pharmazeutischen Industrie sowie in der Humanmedizin einsetzbar.

Eine antikonvulsive Wirkung von 3-Aminopyrrolen ist bisher nicht bekannt. Es ist beschrieben, daß 3-Aminopyrrole, die in 4-Stellung eine Aminocarbonylgruppe (DE 2 605 419) oder eine Carbonylgruppe (US 4 198 502) tragen, als ZNS-wirksame Substanzen eingestuft wurden. Diese Wirkung ist konkret als sedierend und analgetisch benannt, aber durch keinerlei Testergebnisse belegt. Die Synthese dieser Verbindungen erfolgte durch Modifizierung von Aminopyrrolderivaten, die ihrerseits aus α-Aminonitrilen und β-Dicarbonylverbindungen gewonnen wurden (DE 2 605 419, DE 2 439 284, DE 2 462 967, DE 2 462 966, DE 2 462 963, GB 1 492 663, US 4 198 502). Sechs Vertreter von 3-Morpholino-4-arylpyrrolcarbonsäureestern mit einem stark eingegrenzten Substituentenmuster sind durch Cyclisierung von 3-Alkoxycarbonylmethylamino-2-arylthioacrylsäuremorpholiden hergestellt worden (A. Knoll, J. Liebscher Khim. Geterotsikl. Soedin. 5, 1985, 628). Über eine pharmakologische Wirkung derartiger Verbindungen ist bisher nichts bekannt. 3-Amino-4-arylpyrrole, deren Aminogruppe jedoch unsubstituiert ist, wurden durch Reduktion zugehöriger 3-Nitropyrrole gewonnen (J. M. Tedder, B. Webster J. Chem. Soc. 1960, 3270).
3-Amino-2,4-diphenylpyrrol entsteht bei der Kondensation von Phenacylamin mit sich selbst [S. Gabriel Ber. dtsch. Chem. Ges. 41 (1908)1127].
Die bekannten Verfahren beschreiben keine an der Aminogruppe substituierten 3-Amino-4-arylpyrrole, die eine antikonvulsive Wirkung besitzen. Die Substituentenvariabilität der bekannten Verfahren ist stark eingeschränkt.

Die bekannten Antikonvulsiva besitzen den Nachteil unerwünschter Nebenwirkungen (z.B. Neurotoxizität).

In EP 249 236 sind 3-Aminopyrrole beschrieben, die in 2-Stellung eine Tetrazol-5-yl-aminocarbonylgruppe aufweisen, wobei die 3-Aminogruppe mit niederem Alkyl substituiert sein kann. Diese Derivate werden durch Umsetzung der entsprechenden 2-Carboxy-Verbindung mit 5-Aminotetrazol hergestellt. Von diesen Verbindungen ist angegeben, daß sie antiallergische und antiinflammatorische Wirksamkeit besitzen. Eine antikonvulsive Wirkung ist nicht genannt.

Aufgabe der Erfindung ist die Entwicklung neuer, ZNS-wirksamer 3-Aminopyrrole , insbesondere solcher, die antikonvulsive oder analgetische Eigenschaften besitzen, die Entwicklung von Verfahren zu ihrer Herstellung und ihre Verwendung als Arzneistoffe oder als Arzneimittel. Weitere Aufgabe ist, die Verwendung einiger als Antiallergika bzw. entzündungshemmende Wirkstoffe bekannter 3-Aminopyrrole für das obige Indikationsgebiet anzugeben. Dabei wird angestrebt, geringere Nebenwirkungen, z. B. eine geringere Neurotoxizität, zu erreichen als bei den derzeitig üblichen Antikonvulsiva.

Die Aufgabe wird gemäß den unabhängigen Ansprüchen gelöst. Die abhängigen Ansprüche betreffen vorteilhafte Ausführungsformen.

Überraschenderweise wurde gefunden, daß bestimmte 3-Aminopyrrole pharmakologisch wertvolle Eigenschaften, vorzugsweise antikonvulsive und auch analgetische Wirkung, besitzen, welche die allgemeine Formel I aufweisen, 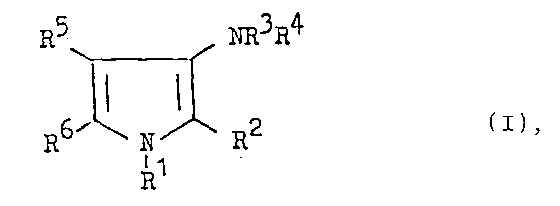 in der die Substituenten R¹ bis R⁶ die den nachstehenden Kombinationen entsprechenden Bedeutungen besitzen:

| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |

Weiterhin verden erfindungsgemäß Verfahren zur Herstellung von 3-Aminopyrrolen der allgemeinen Formel I angegeben.

Die Substituenten R¹ bis R⁶ sind dabei jeweils wie oben definiert.

Die erfindungsgemäßen Verfahren zur Herstellung der 3-Aminopyrrole der
Formel I
sind dadurch gekennzeichnet, daß
(1) ein Aminoacrylsäurederivat der allgemeinen Formel II 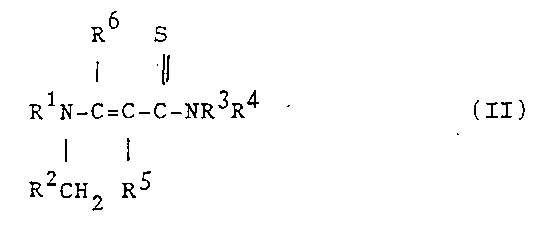 cyclisiert wird,
   vorzugsweise in Gegenwart eines Alkylierungsmittels, eines Oxidationsmittels oder eines Dehydratisierungsmittels und vorzugsweise in Gegenwart einer Base;
(2) zur Herstellung von Verbindungen der Formel I, in der R¹ und R⁶ Wasserstoff und NR³R⁴ Morpholino bedeuten,
   ein Aminoacrylsäurederivat der allgemeinen Formel III, 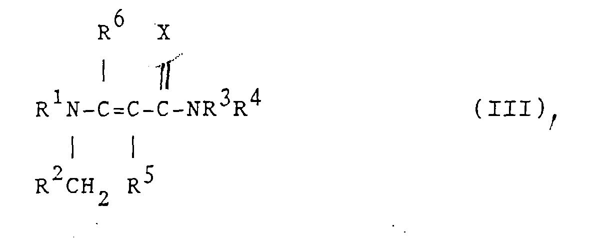 in der X Sauerstoff oder ein substituiertes Stickstoffatom bedeutet,
   gegebenenfalls in Gegenwart eines Alkylierungsmittels oder eines Dehydratisierungsmittels und vorzugsweise in Gegenwart einer Base,
   cyclisiert wird;
(3) ein Trimethiniumsalz der allgemeinen Formel IV, 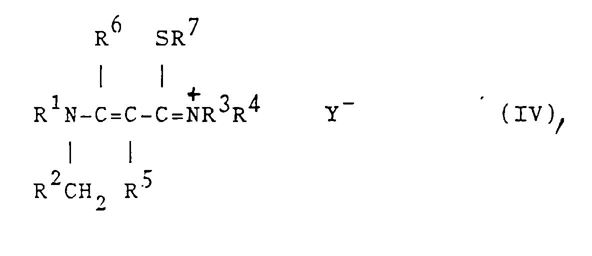 in der R⁷ eine Alkyl- oder Aralkylgruppe und Y⁻ ein Säurerestanion, wie beispielsweise ein Halogenid, Perchlorat, Alkylsulfat, Sulfonat, Sulfat, Tetrafluor- oder Tetraarylborat oder Picrat, bedeuten, oder ggfs. die zugehörige freie Base eines Trimethiniumsalzes der allgemeinen Formel IV cyclisiert wird, vorzugsweise mit einer Base;
(4) zur Herstellung von Verbindungen der Formel I, in der R¹ und R⁶ Wasserstoff und NR³R⁴ Morpholino bedeuten,
   ein Trimethiniumsalz der allgemeinen Formel V, 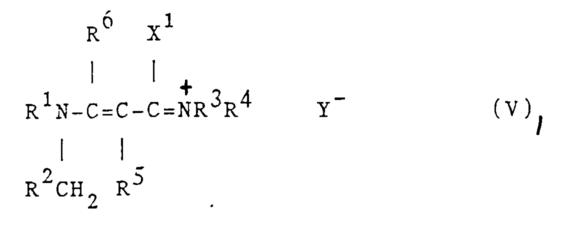 in der X¹ eine Abgangsgruppe, wie beispielsweise ein Halogen, eine Alkoxy- oder eine Aminogruppe, und Y⁻ ein Säurerestanion, beispielsweise ein Halogenid, Perchlorat, Alkylsulfat, Sulfonat, Sulfat, Tetrafluor- oder Tetraarylborat oder Picrat, bedeuten,
   oder ggfs. die zugehörige freie Base eines Trimethiniumsalzes der allgemeinen Formel V cyclisiert wird, vorzugsweise in Gegenwart einer Base;
(5) zur Herstellung von Verbindungen der Formel I, in der R¹ und R⁶ Wasserstoff und NR³R⁴ Morpholino bedeuten,
   ein Acrylsäureamidderivat der allgemeinen Formel VI, 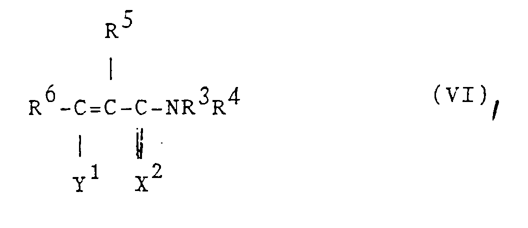 in der X² ein Sauerstoff-, Schwefel- oder ein mono- oder disubstituiertes Stickstoffatom und Y¹ eine Abgangsgruppe, wie beispielsweise eine substituierte oder unsubstituierte Aminogruppe, eine Alkylthio-, eine Alkoxy-, eine Hydroxy-, eine Mercapto- oder eine Acyloxygruppe oder ein Halogen, bedeuten,
   mit einem Amin der allgemeinen Formel VII

   R²CH₂NHR¹ (VII)

   umgesetzt wird,
   vorzugsweise mit einem Alkylierungsmittel oder einem Oxidationsmittel und vorzugsweise mit einer Base;
(6) zur Herstellung von Verbindungen der Formel I, in der R¹ und R⁶ Wasserstoff und NR³R⁴ Morpholino bedeuten,
   ein Iminiumsalz der allgemeinen Formel VIII, 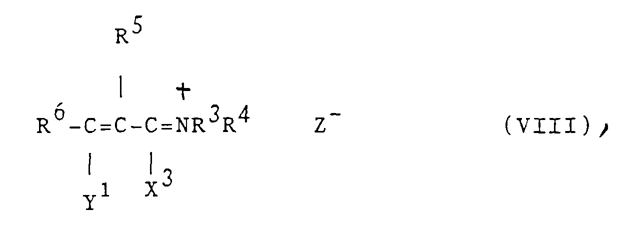 in der X³ und Y¹ gleich oder verschieden sind und eine Abgangsgruppe, wie beispielsweise eine substituierte oder unsubstituierte Aminogruppe, eine Alkylthio-, eine Alkoxy-, eine Hydroxy-, eine Mercapto-, eine Acyloxygruppe oder ein Halogen, und Z⁻ ein Säurerestanion, beispielsweise ein Halogenid, Perchlorat, Alkylsulfat, Sulfonat, Sulfat, Tetrafluor- oder Tetraarylborat oder Picrat, bedeuten,
   mit einem Amin der allgemeinen Formel VII wie oben definiert und mit einer Base umgesetzt wird;
(7) zur Herstellung von Verbindungen der Formel I, in der R¹ Methyl, Ethyl, Benzyl oder Ethoxycarbonylmethyl bedeutet,
   ein 1-unsubstituiertes 3-Aminopyrrol der allgemeinen Formel IX 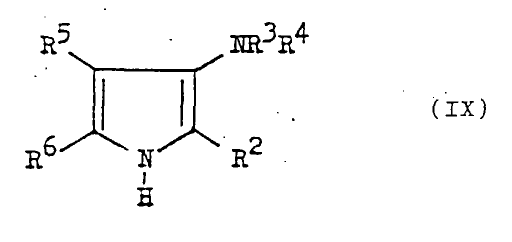 mit einem Alkylierungsmittel der allgemeinen Formel X,

   R¹-Y² (X),

   in der Y² eine Abgangsgruppe, wie beispielsweise ein Halogen, eine Sulfonyloxy- oder eine Diazogruppe, bedeuten, und einer Base umgesetzt wird;
(8) zur Herstellung von Verbindungen der Formel I, in der R² COZ¹ mit Z¹ = Hydroxy, NaO, Methoxy, Ethoxy oder Anilino bedeutet,
   ein Pyrrolcarbonsäurederivat der allgemeinen Formel XI, 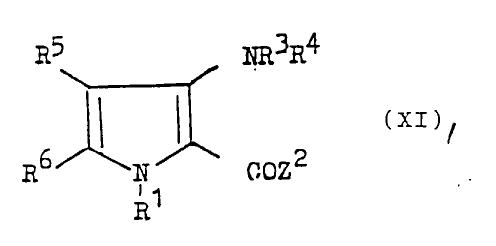 in der Z² verschieden von Z¹ eine Abgangsgruppe, wie beispielsweise eine Hydroxy-, Alkoxy-, Aryloxy-, Acyloxy- oder Alkylthiogruppe oder ein Halogen, bedeutet,
   mit einem nucleophilen Reagens der allgemeinen Formel XII

   Z¹H (XII)

   mit Z¹ wie oben
   oder der deprotonierten Form eines nucleophilen Reagens der allgemeinen Formel XII
   und ggfs. mit einer Säure oder Base umgesetzt wird;
(9) zur Herstellung von Verbindungen der Formel I, in der R¹ und R⁶ Wasserstoff und NR³R⁴ Morpholino bedeuten,
   ein Enamin der allgemeinen Formel XIII, 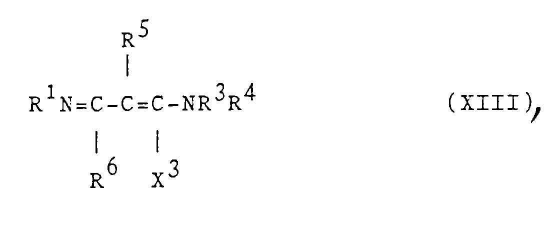 in der X³ eine Abgangsgruppe, wie beispielsweise eine Alkoxy-, Alkylmercapto- oder substituierte Aminogruppe oder ein Halogen, bedeutet,
   oder ein Salz eines Enamins der allgemeinen Formel XIII, wie beispielsweise ein Hydrohalogenid, ein Hydroperchlorat, ein Hydrosulfonat oder ein Hydrotetrafluorborat,
   mit einem Alkylierungsmittel der allgemeinen Formel XIV,

   R²CH₂Z³ (XIV),

   in der Z³ eine Abgangsgruppe, wie beispielsweise ein Halogen, ein Sulfonat, ein Alkylsulfat oder ein Trifluoracetat, bedeutet,
   und einer Base umgesetzt wird;
(10) zur Herstellung von Verbindungen der Formel I, in der R¹ und R⁶ Wasserstoff und NR³R⁴ Morpholino bedeuten,
   ein Enamin der allgemeinen Formel XV 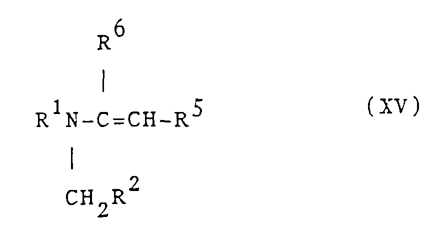 mit einem Iminiumsalz der allgemeinen Formel XVI, 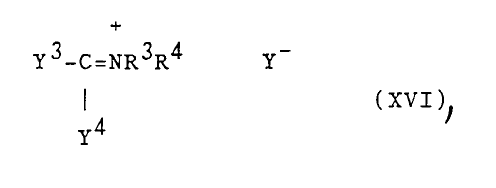 in der Y³ und Y⁴ gleiche oder verschiedene Abgangsgruppen, wie beispielsweise Chlor, Aminogruppen, Alkylmercapto- oder Alkoxygruppen, und Y⁻ein Säurerestanion, beispielsweise ein Halogenid, ein Sulfonat, ein Sulfat oder ein Trifluoracetat, darstellen,
   und ggfs. mit einer Base umgesetzt wird;
(11) zur Herstellung von Verbindungen der Formel I, in der R² R⁷C=Z⁴ bedeutet, wobei R⁷ Methyl, Methoxy, Ethoxy oder
   NH-C₆H₅ darstellt und Z⁴ für ein Sauerstoffatom steht,
   ein 2-unsubstituiertes 3-Aminopyrrol der allgemeinen Formel XVII 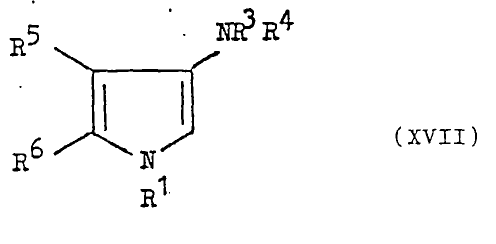 mit einer Carbonylverbindung der allgemeinen Formel XVIII 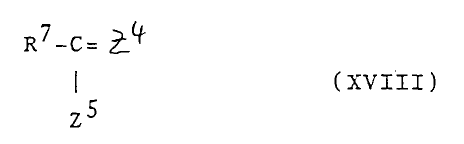 mit R⁷ und Z⁴ wie oben, in der Z⁵ eine Abgangsgruppe, wie beispielsweise ein Halogen oder eine Acyloxygruppe, bedeutet,
   oder, sofern bei den Verbindungen der Formel I mit R²= R⁷C-Z⁴ R⁷ NHC₆H₅ bedeutet,
   auch mit einem Heterocumulen der allgemeinen Formel XIX

   R⁵N=C=Z⁴ (XIX)

   mit Z⁴ wie oben
   und ggfs. mit einer Lewis- oder Protonsäure umgesetzt wird;
(12) zur Herstellung von Verbindungen der Formel I, in der R¹ und R⁶ Wasserstoff und NR³R⁴ Morpholino bedeuten,
   ein 3-substituiertes Pyrrol der allgemeinen Formel XX 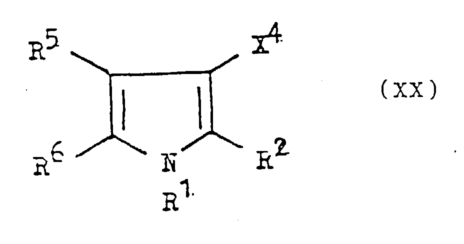 in der X⁴ eine Abgangsgruppe, wie ein Halogen, eine Hydroxy- oder Alkoxygruppe, eine Alkylthiogruppe oder eine Diazoniumgruppe, bedeutet,
   mit einem Amin der allgemeinen Formel XXI

   HNR³R⁴ (XXI)

   umgesetzt wird;
(13) zur Herstellung von Verbindungen der Formel I, in der R² COOR⁷ bedeutet, wobei R⁷ Methyl oder Ethyl darstellt,
   eine 3-Aminopyrrolcarbonsäure der allgemeinen Formel XXII 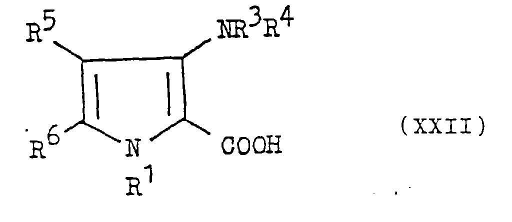 oder ein Salz einer 3-Aminopyrrolcarbonsäure der allgemeinen Formel XXII, beispielsweise ein Natrium-, Kalium- oder Ammoniumsalz, mit einem Alkylierungsmittel der allgemeinen Formel XXIII

   R⁷X⁵ (XXIII)

   mit R⁷ wie oben, in der X⁵ eine Abgangsgruppe, beispielsweise ein Halogen oder eine Sulfonylgruppe, bedeutet,
   und ggfs. mit einer Base umgesetzt wird.

Beim erfindungsgemäßen Verfahren, Varianten 1 bis 9, werden als Base vorzugsweise ein Amin, ein Alkali- oder Erdalkalihydroxid oder -hydrid, ein Alkalicarbonat oder ein Metallamid verwendet.

Bei den Varianten 1, 2 und 5 werden als Alkylierungsmittel bevorzugt ein Alkyl- oder Aralkylhalogenid, ein Alkylsulfonat, ein Dialkylsulfat, ein Trialkyloxoniumsalz oder Diazomethan verwendet.

Bei den Varianten 1 und 5 werden als Oxidationsmittel vorzugsweise Wasserstoffperoxid, eine Persäure oder ein Persulfat verwendet.

Die erfindungsgemäßen pharmazeutischen Zusammensetzungen enthalten ein oder mehrere 3-Aminopyrrole
als Wirkstoffe neben einem oder mehreren physiologisch verträglichen Hilfs- und/oder Trägerstoffen und ggfs. einem Verdünnungsmittel,
wobei die 3-Aminopyrrole unter den Verbindungen der Formel I, wie sie in Anspruch 1 definiert sind, sowie weiteren Verbindungen der Formel I wie in Anspruch 1 mit den nachstehenden Kombinationen der Substituenten R¹ bis R⁶ ausgewählt sind:

| R¹ | R² | R³ | R⁴ | R⁵ | R⁶ |
|---|---|---|---|---|---|
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |

Die erfindungsgemäßen pharmazeutischen Zusammensetzungen, insbesondere mit ZNS-Wirksamkeit und vorzugsweise antikonvulsiver und/oder analgetischer Wirkung, enthalten.

Aminopyrrole der Formel I wie in Anspruch 6 definiert vorzugsweise in einer Menge von insgesamt 0,5 bis 20 mg/kg pro Einzeldosis.

Die Erfindung betrifft entsprechend auch die Verwendung der 3-Aminopyrrole der Formel I nach Anspruch 1 sowie der in Anspruch 6 aufgeführten weiteren Verbindungen der Formel I als oder zur Herstellung von pharmazeutischen Mitteln zur Behandlung von Erkrankungen des Zentralnervensystems, insbesondere von Epilepsien verschiedener Formen, nichtepileptischen Anfällen, Migräne und Schmerzen verschiedener Genese.

Das Verfahren zur Herstellung der pharmazeutischen Zusammensetzungen ist dadurch gekennzeichnet, daß der oder die Wirkstoffe unter Verwendung pharmazeutisch geeigneter Hilfs- und/oder Trägerstoffe und/oder Verdünnungsmittel in an sich bekannter Weise in eine galenisch geeignete Form gebracht werden.

Die erfindungsgemäßen Verbindungen sind neu. Die erfindungsgemäßen Verbindungen bzw. die im den erfindungsgemäßen pharmazeutischen Mitteln verwendeten Verbindungen der Formel I zeigen im Test in verschiedenen Krampfmodellen eine hohe antikonvulsive Wirkung und zeichnen sich durch geringe Toxizität und vor allem einen wesentlich höheren protektiven Index aus als derzeit bekannte handelsübliche Antikonvulsiva. Die antikonvulsive Wirkung ist überraschend, da bisher generell bei 3-Aminopyrrolen keine solche Wirkung beschrieben ist. Die neuen Wirkstoffe können in bekannter Weise in übliche pharmazeutische Formulierungen überführt werden, wie beispielsweise Tabletten, Kapseln, Dragees, Granulate oder Lösungen, unter Verwendung inerter, nicht-toxischer, pharmazeutisch geeigneter Trägerstoffe oder Lösungsmittel.

Die Erfindung wird nachstehend an einigen Ausführungsbeispielen erläutert.

### Beispiel 1

Synthese von 3-Aminopyrrolen der Formel I. Die nach den verschiedenen Varianten hergestellten 3-Aminopyrrole der allgemeinen Formel I sind in der Tabelle zusammengestellt.

### Variante A

Eine Lösung von 10 mmol Aminoacrylsäurederivat der allgemeinen Formel II und 1,9 g Dimethylsulfat oder 2,2 g Methyliodid in 20 ml Chlorororm wird 15 Minuten unter Rückfluß erhitzt. Nach Abziehen des Lösungsmittels wird mit 10 ml Ethanol oder Methanol verdünnt und der Mischung 1 g Triethylamin zugesetzt. Es wird 5 Minuten zum Sieden erhitzt und nach dem Abkühlen mit etwas Wasser verdünnt. Das 3-Aminopyrrol der allgemeinen Formel I wird abgesaugt und umkristallisiert.

### Variante B

Eine Suspension von 10 mmol Aminoacrylsäurederivat der allgemeinen Formel II in 10 ml Wasser wird tropfenweise unter Kühlung mit 30 mmol 30% Wasserstoffperoxid versetzt, so das die Temperatur unter 20 °C bleibt. Man läßt 30 Minuten bei Raumtemperatur ausreagieren und kühlt dann auf 5°C. Das Festprodukt wird abgesaugt und mit 10 ml Acetonitril und 2 ml Triethylamin versetzt. Die Mischung wird 30 Minuten zum Sieden erhitzt und nach dem Abkühlen mit wenig Wasser verdünnt. Des Endprodukt der allgemeinen Formel I wird abgesaugt und umkristallisiert.

### Variante C

Eine Lösung von 10 mmol Aminoacrylsäurederivat der allgemeinen Formel III mit X = 0 wird mit 10 ml Acetanhydrid und 2 ml Ethyldiisopropylamin versetzt. Man erhitzt die Mischung 2 Stunden unter Rückfluß. Die Reaktionsmischung wird mit überschüssigem Wasser hydrolysiert, das 3-Aminopyrrol der allgemeinen Formel I abgesaugt und umkristallisiert.

### Variante D

10 mmol Trimethiniumsalz der allgemeinen Formel IV mit R⁷ = Methyl und Y⁻ = I⁻ werden mit 10 ml Ethanol und einer Lösung von 0,4 g Natrium in 5 ml Ethanol versetzt. Es wird 30 Minuten auf dem siedenden Wasserbad erhitzt. Nach dem Abkühlen wird unter Kühlung mit Eisessig abgestumpft und mit wenig Wasser verdünnt. Das Endprodukt der allgemeinen Formel I wird abgesaugt und umkristallisiert.

### Variante E

Eine Lösung von 10 mmol eines Trimethiniumsalzes der allgemeinen Formel V mit X¹ = Cl und Y = Cl⁻ in 11 ml Ethanol wird mit 1 ml 1,8-Diazabicyclo[5.4.0]-undecen versetzt und 15 Minuten auf 50°C erwärmt. Das beim Abkühlen ausfallende 3-Aminopyrrol der allgemeinen Formel I wird abgesaugt, mit etwas Wasser gewaschen und umkristallisiert.

### Variante F

Eine Lösung von 10 mmol Trimethiniumsalz der allgemeinen Formel V mit X¹ = OEthyl und Y⁻ = BF₄⁻ in 10 ml Acetonitril wird mit 1 g Kaliumcarbonat versetzt und 20 Minuten unter Rückfluß erhitzt. Nach dem Abkühlen wird die Reaktionsmischung unter Kühlung mit verdünnter Salzsäure neutralisiert. Es wird abgesaugt und umkristallisiert.

### Variante G

10 mmol eines Acrylsäureamidderivates der allgemeinen Formel VI mit X² = S und Y¹ = Dimethyiamino in Form seines Hydroperchlorates werden mit 15 ml Methanol, 10 mmol eines Amins der allgemeinen Formel VII und 3 g Triethylamin versetzt. Die Mischung wird 5 Minuten zum Sieden erhitzt, Nach dem Abkühlen wird mit etwas Eiswasser verdunnt. Der ausfallende Niederschlag wird abgesaugt, getrocknet, mit 10 ml Ethanol und 2 g p-Toluolsulfonsäuremethylester versetzt und 10 Minuten unter Rückfluß erhitzt. Nach Zugabe von 2 ml Triethylamin wird weitere 10 Minuten unter Rückfluß erhitzt. Fällt beim Abkühlen kein Endprodukt aus, wird mit wenig kaltem Wasser verdünnt. Es wird abgesaugt und umkristallisiert.

### Variante H

Es wird analog der Variante G verfahren, wobei jedoch ein Acrylsäureamidderivat der allgemeinen Formel VI mit X¹ = S und Y¹ = OH verwendet und das Amin der allgemeinen Formel VII als Hydrochlorid eingesetzt wird.

### Variante I

Eine Mischung von 10 mmol eines Iminiumsalzes der allgemeinen Formel VIII mit X³ = Methylmercapto, Y¹ = Dimethylamino- und Z⁻ = I⁻ mit 15 ml Ethanol wird mit 10 mmol eines Amins der allgemeinen Formel VII versetzt und kurz zum Sieden erhitzt. Nach Zugabe von 2 ml Triethylamin wird 30 Minuten unter Rückfluß erhitzt. Nach dem Abkühlen wird gegebenenfalls mit etwas Wasser verdünnt, das Endprodukt der allgemeinen Formel I abgesaugt und umkristallisiert.

### Variante J

Es wird analog Variante I verfahren, wobei jedoch ein Iminiumsalz der allgemeinen Formel VIII mit Y¹ = Morpholino, X³ = Chlor und Z⁻ = Cl⁻ verwendet wird.

### Variante K

Eine Lösung von 5 mmol 3-Aminopyrrol der allgemeinen Formel IX in 40 ml Methylenchlorid wird mit 7,5 mmol Alkylierungsmittel der allgemeinen Formel X mit Y² = Br oder I und 0,05 g Benzyltriethylammoniumbromid versetzt. Nach Zugabe einer Lösung von 0,05 g Benzyltriethylammoniumbromid in 15 ml 50% Natronlauge wird die Mischung 5 Stunden bei Raumtemperatur kräftig gerührt. Danach wird mit verdünnter Salzsäure neutralisiert, die organische Phase abgetrennt und die wäßrige Phase zweimal mit Methylenchlorid extrahiert. Die vereinigten organischen Extrakte werden mit Wasser und Kaliumcarbonatlösung gewaschen. Nach dem Trocknen über Natriumsulfat wird das Lösungsmittel abgedampft. Das Endprodukt der allgemeinen Formel I kristallisiert bei Zugabe von wenig Methanol oder Cyclohexan.

### Variante L

10 mmol 3-Aminopyrrol der allgemeinen Formel IX werden in 50 ml Tetrahydrofuran gelöst. Zu der auf 0 °C gekühlten Lösung werden portionsweise unter Rühren 11 mmol Natriumhydrid gegeben. Danach werden 11 mmol Alkylierungsmittel der allgemeinen Formel X mit Y² = Cl Zugegeben. Die Mischung wird 1 Stunde bei Raumtemperatur stehengelassen und anschließend 1,5 h unter Rückfluß erhitzt. Nach dem Abkühlen wird abfiltriert und das Filtrat eingeengt. Aus dem Rückstand kristallisiert bei Zugabe von wenig Ethanol oder Cyclohexan das Endprodukt der allgemeinen Formel I aus. Es wird abgesaugt und umkristallisiert.

### Variante M

Eine Mischung von 3 mmol Pyrrolcarbonsäurederivat der allgemeinen Formel XI mit Z² = OEthyl, 20 ml Ethanol und 8 ml 2N Natronlauge (entspricht der deprotonierten Form des nucleophilen Reagens der allgemeinen Formel XII mit Z¹ = OH) wird 2 Stunden bei Raumtemperatur stehengelassen und anschließend eine Stunde auf dem siedenden Wasserbad erwärmt. Nach dem Erkalten kristallisiert das Endprodukt der allgemeinen Formel I mit R²-COONa aus. Dieses wird abgesaugt. Es kann durch Zugabe von Salzsäure zu seiner ethanolisch-wäßrigen Lösung in die entsprechende freie Säure der allgemeinen Formel I mit R² = COOH überführt werden.

### Variante N

Eine Lösung von 20 mmol Pyrrolcarbonsäurederivat der allgemeinen Formel XI mit Z² = Methoxy in 50 ml eines Nucleophils der allgemeinen Formel XII mit Z¹ = Ethoxy wird mit einer Lösung von 0,23 g Natrium in 7 ml Ethanol versetzt und 2 Stunden erhitzt, wobei das Lösungsmittel allmählich abdestilliert. Man bricht die Destillation bei etwa 6 ml Destillationsrückstand ab und läßt abkühlen. Der Niederschlag wird abgesaugt, mit wenig kaltem Ethanol gewaschen und umkristallisiert.

### Variante O

Eine Lösung von 10 mmol Pyrrolcarbonsäurederivat der allgemeinen Formel XI mit Z² = Chlor in 8 ml Acetonitril wird mit 10 mmol des nucleophilen Reagens der allgemeinen Formel XII mit Z¹ = PhenylNH und 1 ml Triethylamin versetzt. Nach Abklingen der exothermen Reaktion wird kurz zum Sieden erhitzt. Das beim Abkühlen auskristallisierende Endprodukt der allgemeinen Formel I wird abgesaugt und umkristallisiert.

### Variante P

Zu einer Mischung von 2.8 g Kaliumcarbonat und 20 ml DMF werden 10 mmol des Enaminderivates der allgemeinen Formel XIII mit X³ = Methylmercapto und 12 mmol Alkylierungsmittel der allgemeinen Formel XIV mit Z³ = Cl gegeben. Es wird 3 Stunden bei 100 °C gerührt. Nach Zugabe von 2 ml Triethylamin wird noch 2 Stunden bei 85 °C gerührt. Nach dem Abkühlen wird auf Eiswasser gegossen. Das Endprodukt wird abgesaugt und umkristallisiert.

### Variante Q

Eine Lösung von 10 mmol Enamin der allgemeinen Formel XV in 10 ml Acetonitril wird mit 10 mmol Iminiumsalz der allgemeinen Formel XVI mit Y³ = Y⁴ = Cl und Y⁻ = Cl⁻ versetzt. Man erwärmt 30 Minuten auf dem siedenden Wasserbad und gibt dann 3 ml Triethylamin zu. Es wird 30 Minuten unter Rückfluß erhitzt. Nach dem Abkühlen wird auf Eis gegossen. Das Endprodukt der allgemeinen Formel I wird abgesaugt und umkristallisiert.

### Variante R

10 mmol 3-Aminopyrrol der allgemeinen Formel XVII werden in 10 ml Acetonitril gelöst, und mit 20 mmol Carbonylverbindung der allgemeinen Formel XVIII mit Z⁴ = 0 und Z⁵ = Chlor versetzt. Die Mischung wird 20 Minuten unter Rückfluß erhitzt. Nach dem Abkühlen wird auf Eis gegossen. Das Endprodukt der Formel I mit R² = COR⁷ wird abgesaugt und umkristallisiert.

### Variante S

Es wird analog Variante R verfahren, jedoch unter Verwendung einer Carbonylverbindung der allgemeinen Formel XVIII mit Z⁴ = 0 und Z⁵ = OCOCH-.

### Variante T

10 mmol Pyrrol der allgemeinen Formel XVII werden in 10 ml Benzol gelöst. Man gibt 1o mmol eines Heterocumulens der allgemeinen Formel XIX mit Z⁴ = 0 zu und erwärmt 40 Minuten unter Rückfluß. Das Lösungsmittel wird abgedampft und der Rückstand umkristallisiert.

### Variante U

10 mmol eines 3-substituierten Pyrrols der allgemeinen Formel XX mit X⁴ = Methylthio werden in 10 ml Methanol oder Ethanol gelöst und mit 10 mmol Amin der allgemeinen Formel XXI versetzt. Es wird 30 Minuten unter Rückfluß erhitzt. Danach wird ein Teil des Lösungsmittels abgedampft, das Endprodukt der allgemeinen Formel I abgesaugt und umkristallisiert.

### Variante V

Es wird analog Variante U verfahren, jedoch unter Verwendung eines 3-substituierten Pyrrols der allgemeinen Formel XX mit X⁴ = Chlor.

### Variante W

10 mmol des Natriumsalzes einer 3-Aminopyrrolcarbonsäure der allgemeinen Formel XXII werden mit 10 ml Acetonitril, 10 mmol Alkylierungsmittel der allgemeinen Formel XXIII mit X⁵ = Br, 1 mmol Methyltrioctylammoniumchlorid und 10 ml Wasser versetzt. Die Mischung wird 4 Stunden unter Rückfluß erhitzt. Nach dem Abkühlen wird die organische Phase abgetrennt. Nach Ausäthern der wäßrigen Phase werden die vereinigten organischen Phasen getrocknet und eingedampft. Das zurückbleibende Endprodukt wird abgesaugt und umkristallisiert.

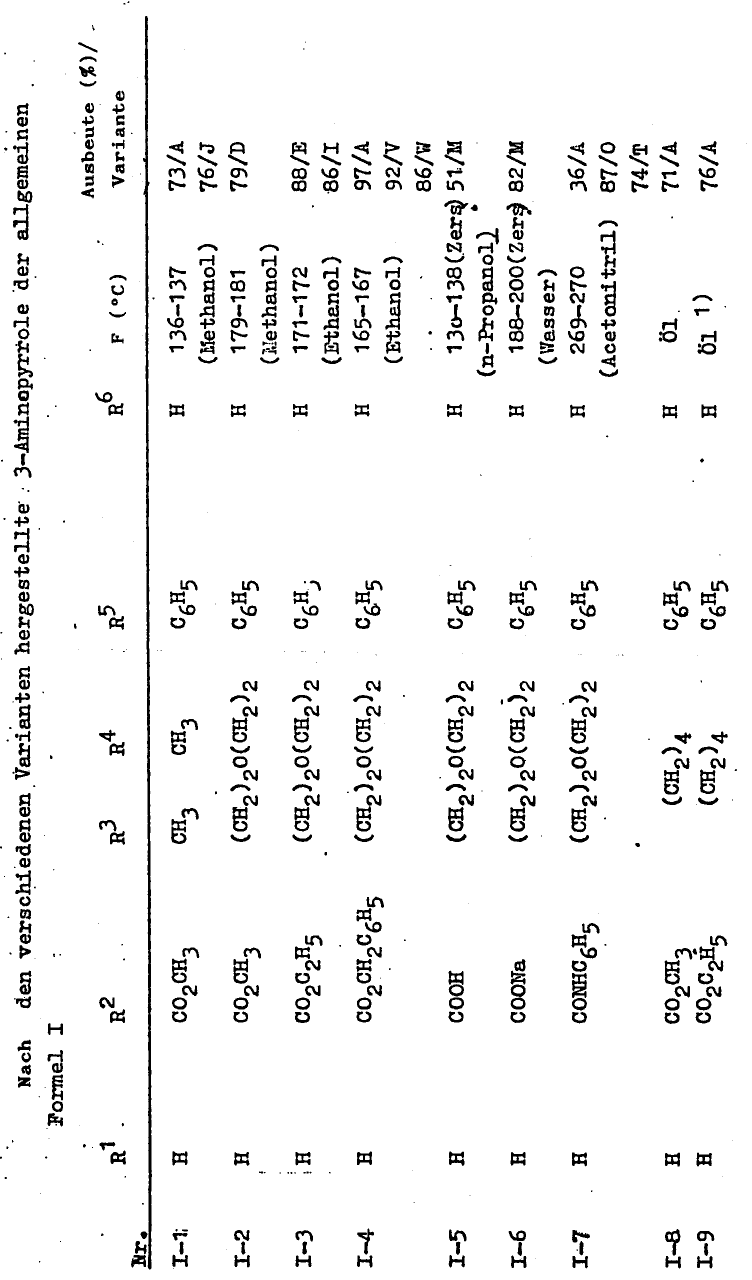

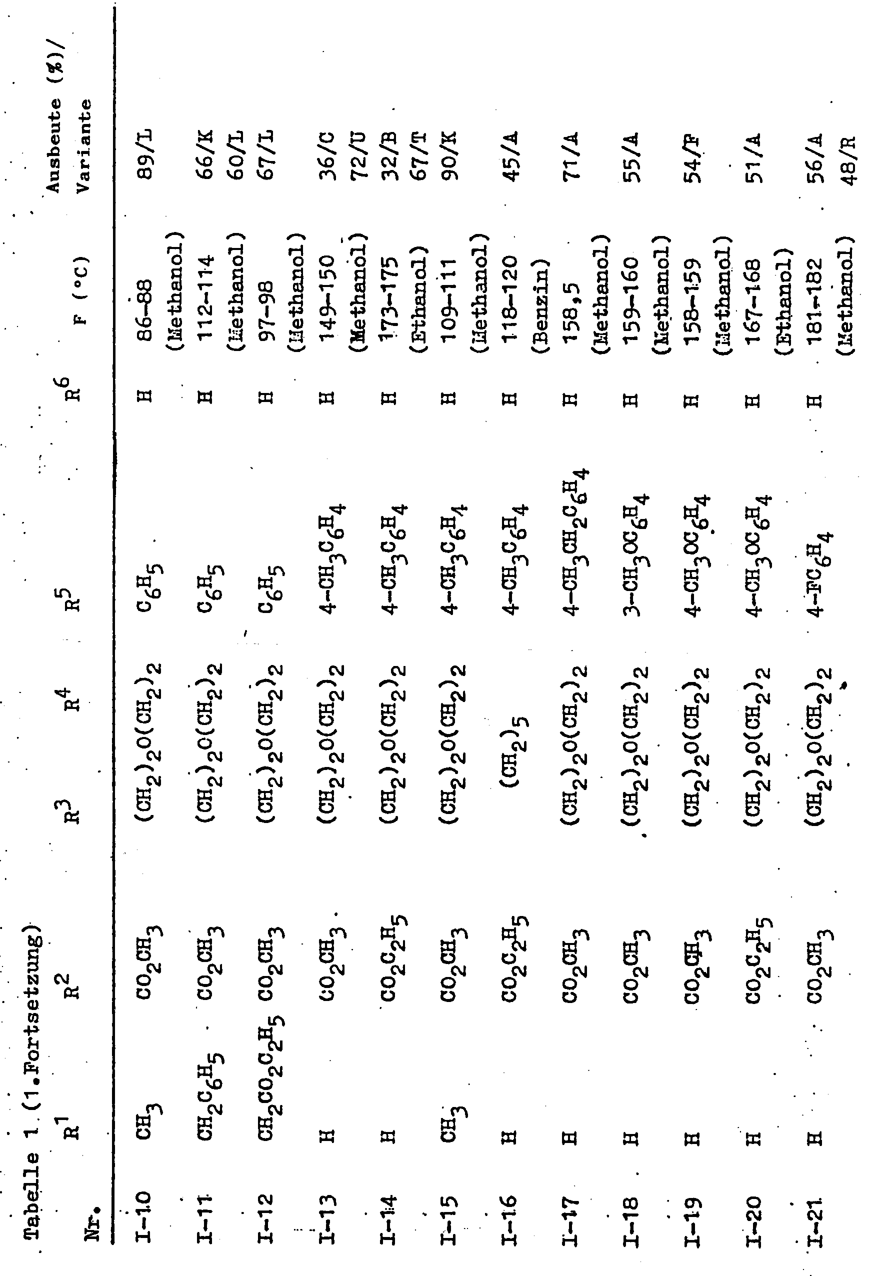

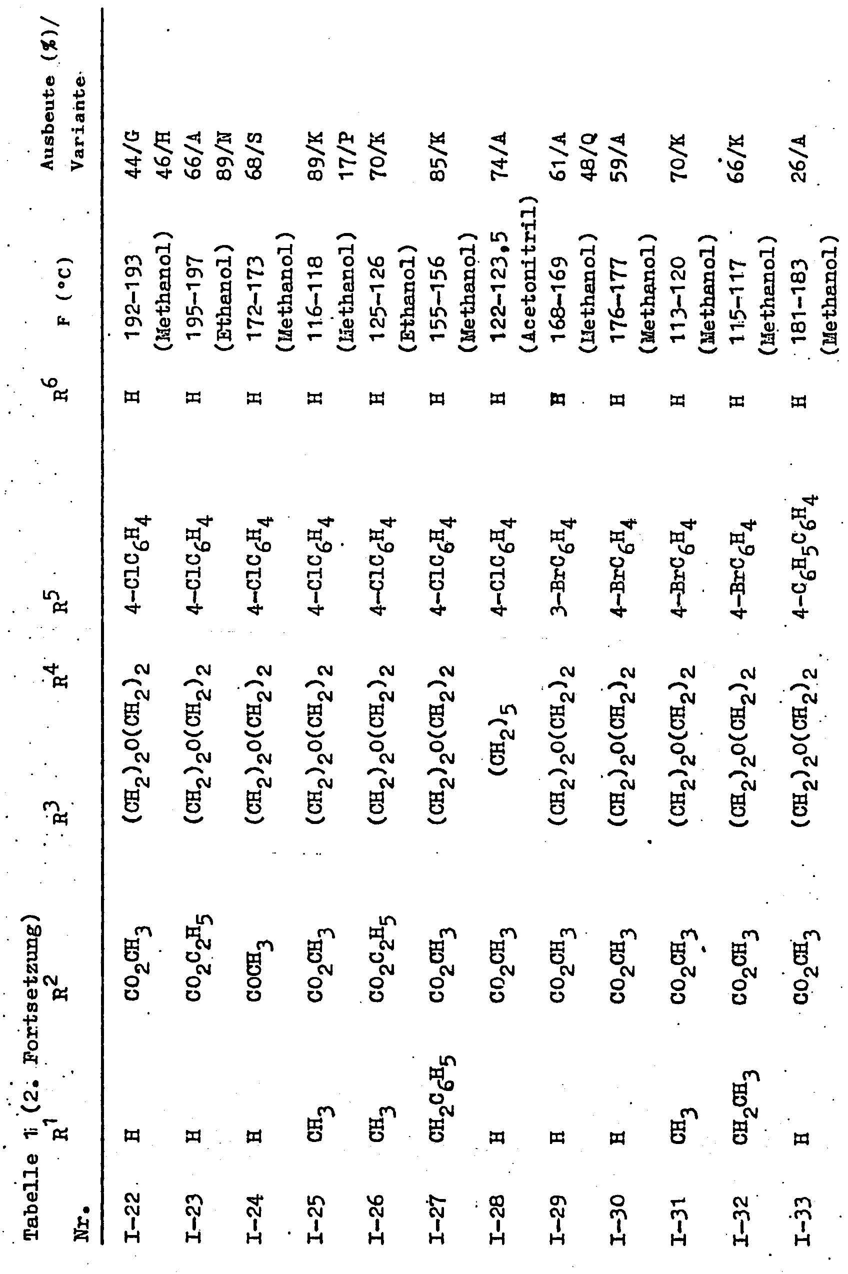

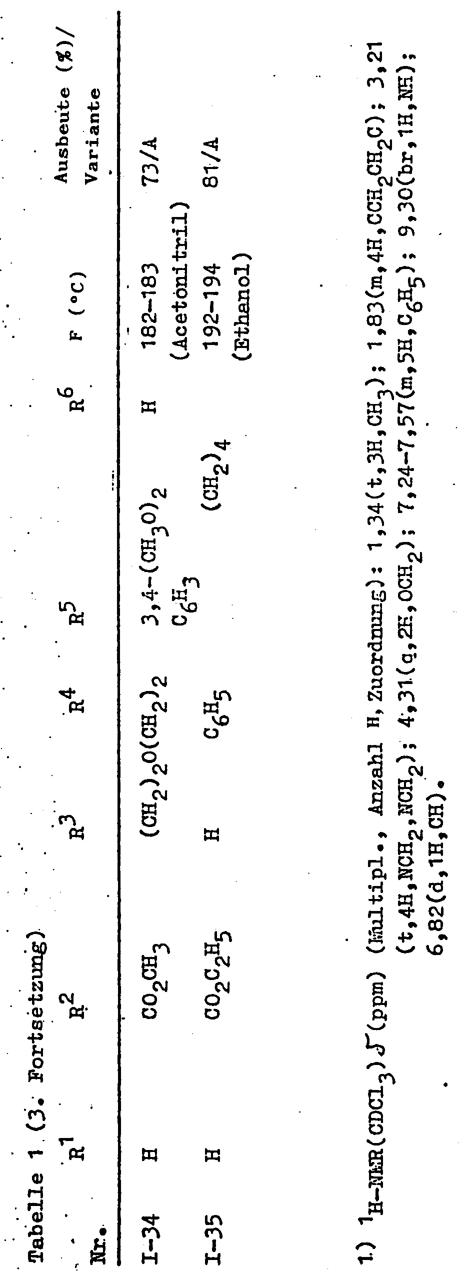

### Beispiel 2

### Bestimmung des Schutzes gegen den maximalen Elektrokrampf (MEK)

Durch elektrische Reizung der Vorderpfoten von Mäusen mit einer Körpermasse (KM) von 18 - 22 g mit einem Reizstromgerät (TUR, Typ RS 12, Impulsfrequenz 35 Hz, Impulsbreite 20 ms, Tastverhältnis 1:1, Gruppendauer zwischen 400 und 600 ms, Stromstärke der Rechteckimpulse 50 mA) wird ein Streckkrampf der Hinterextremitäten ausgelöst.

Antikonvulsiva schützen die Tiere vor dem maximalen Elektrokrampf.

### Ergebnisse:

- Verbindung I-22:: Bei i.p.-Gabe: E_{D50} = 3,9·10⁻⁵ mol/kg
bei p.o.-Gabe: E_{D50} = 4,5·10⁻⁵ mol/kg
- Verbindung I-8 :: Bei i.p.-Gabe: 5·10⁻⁴ mol/kg:70 %
- Vergleichswerte mit Carbamazepin:: Bei i.p.-Gabe: L_{D50} = 4,3·10⁻⁵ mol/kg.

### Beispiel 3

### Bestimmung der Wirkung im pentetrazolinduzierten Krampf

Durch intravenöse Injektion in die Schwanzvene von Mäusen (KM 18 - 22 g) tritt sofort ein Streckkrampf der Hinterextremitäten auf. Die Unterdrückung dieses Krampfbildes gilt als Kriterium für einen antikonvulsiven Effekt der geprüften Substanzen.

### Ergebnisse:

- Verbindung I-22:: Bei i.p.-Gabe: E_{D50} = 4,5 . 10⁻⁵ mol/kg
bei p.o.-Gabe: E_{D50} = 1,5 . 10⁻⁴ mol/kg.

### Beispiel 4

### Bestimmung der Krampfschwelle

Durch Infusion von 100 mg/kg Pentetrazol (Infusionsgeschwindigkeit 36 ml/h) über die Schwanzvene treten als erstes klonische Krämpfe (myoclonische Zuckungen) bei Mäusen (KM 18 - 22 g) auf. Die Verlängerung der Infusionsdauer (in s) bis zum Auftreten der Krämpfe im Vergleich zu Kontrolltieren gilt als Erhöhung der Pentetrazolkrampfschwelle und somit als antikonvulsiver Effekt der geprüften Substanzen.

### Ergebnisse:

- Verbindung I-12:: i.p. bei 5·10⁻⁴ mol/kg: 20,4 % Erhöhung der Krampfschwelle
- Verbindung I-10:: i.p. bei 5·10⁻⁴ mol/kg: 19,4 % Erhöhung.

### Beispiel 5

### Bestimmung der orientierenden letalen Dosis

Mäuse (KM 18 - 22 g) erhalten die zu prüfenden Substanzen in Dosierungen von 5·10⁻⁴, 10⁻³ und 5·10⁻³ mol/kg KM. 24 h post applicationem wird die Letalität der Tiere bestimmt.

### Ergebnisse:

- Verbindung I-6:: oLD größer als 5·10⁻³ mol/kg
- Verbindung I-22:: oLD größer als 5·10⁻³ mol/kg.

### Beispiel 6

### Bestimmung der analgetischen Wirkung mit dem Hot-Plate-Test

Mäuse (KM 18 - 22 g) werden 30 min nach Gabe der Testsubstanzen auf eine Heizplatte (hot plate) von 56 °C gesetzt, und es wird die Reaktionszeit auf diesen thermischen Schmerzreiz bestimmt. Eine Verlängerung der Reaktionszeit von substanzbehandelten Tieren im Vergleich zu Kontrolltieren wird als analgetischer Effekt gewertet.

### Ergebnisse:

- Verbindung I-2:: p.o. bei 10⁻³ mol/kg: 90 % Hemmung (30 min p.a.).

### Vergleichswert:

- Analgin:: 55 % Hemmung.

### Beispiel 7

### Bestimmung der analgetischen Wirkung mit dem Essigsäure-Writhing-Test

Durch i.p.-Gabe von 0,6 %iger Essigsäure werden bei Mäusen (KM 18 - 22 g) Bauchdeckenkrämpfe (writhings) ausgelöst. Als Maß für die Wirkstärke einer Substanz dient die Reduktion der Zahl der Writhing-Reaktionen behandelter Tiere im Vergleich zur Kontrollgruppe. Neben analgetisch wirksamen Verbindungen senken auch verschiedene ZNS-wirksame Verbindungen die Anzahl der Writhings.

### Ergebnisse:

- Verbindung I-2:: p.o. bei 10⁻³ mol/kg: 71,3 % Hemmung
- Verbindung I-6:: p.o. bei 10⁻³ mol/kg: 84,2 % Hemmung
bei 10⁻⁴ mol/kg: 50,2 % Hemmung.

### Vergleichswert:

- Analgin:: p.o. bei 10⁻⁴ mol/kg: 50 % Hemmung.

### Beispiel 8

### Bestimmung der Neurotoxizität mit dem Drehstabmodell

Trainierte Mäuse (KM 18 - 22 g) werden nach Substanzapplikation für 1 min auf den Drehstab (5 Umdrehungen/min) gesetzt. Als Maß für eine Substanzwirkung gilt das vorzeitige Herunterfallen vom Drehstab. Der protektive Index ergibt sich als Quotient von TD₅₀/ED₅₀ MEK.

### Ergebnis:

- Verbindung I-22:: TD₅₀ = 1,4·10⁻³ mol/kg
protektiver Index = 36.

### Vergleichswert:

- Carbamazapin:: TD₅₀ = 2,2·10⁻⁴ mol/kg
Protektiver Index = 5,1.

### Beispiel 9

### Applikationsformen

### Rezepturen für die Applikation:

### Kapseln

3-Aminopyrrol der allgemeinen Formel I wird in der erforderlichen Menge in Polyethylenglycol suspendiert und in eine Gelatinemischung der Zusammensetzung
- Gelatine: 1 Masseteil
- Glycerin: 5 Masseteile
- Wasser: 2 Masseteile
eingearbeitet.

### Kapseln

Es wird eine Mischung mit folgenden Bestandteilen hergestellt:
- Lactose: 5 Masseteile
- Kartoffelstärke: 5 Masseteile
- Magnesiumstearat: 1 Masseteil.

Diesem Gemisch wird die entsprechende Menge der Substanz der allgemeinen Formel I zugesetzt.

Weitere Zubereitungen als Dragees, Tabletten, Lutschbonbons, Granulate, Pulver, wäßrige Suspensionen, Sirupe udgl. sind im Rahmen der Erfindung ebenso möglich.

## Patentansprüche (Patentansprüche für folgende(n) Vertragsstaat(en): BE, CH, LI, DE, FR, GB, IT, NL, SE)

1. 3-Aminopyrrole der Formel I, 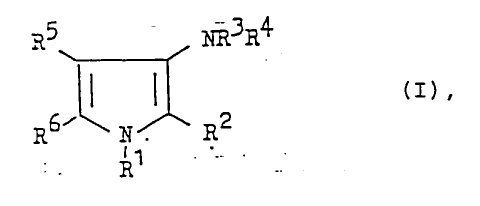 in der die Substituenten R¹ bis R⁶ die den nachstehenden Kombinationen entsprechenden Bedeutungen besitzen:
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |

2. Verfahren zur Herstellung der 3-Aminopyrrole der Formel I nach Anspruch 1,
dadurch gekennzeichnet, daß
(1) ein Aminoacrylsäurederivat der allgemeinen Formel II 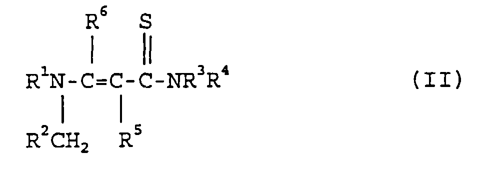 mit R¹ bis R⁶ wie in Anspruch 1
cyclisiert wird,
vorzugsweise in Gegenwart eines Alkylierungsmittels oder eines Oxidationsmittels und vorzugsweise in Gegenwart einer Base;
(2) zur Herstellung von Verbindungen der Formel I, in der R¹ und R⁶ Wasserstoff und NR³R⁴ Morpholino bedeuten,
ein Aminoacrylsäurederivat der allgemeinen Formel III, 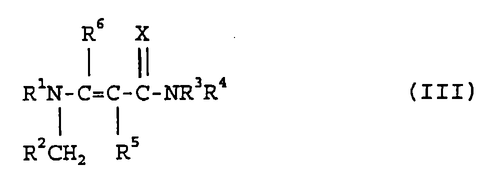 mit R¹ bis R⁶ wie in Anspruch 1, in der X Sauerstoff oder ein substituiertes Stickstoffatom bedeutet,
gegebenenfalls in Gegenwart eines Alkylierungsmittels oder eines Dehydratisierungsmittels und vorzugsweise in Gegenwart einer Base,
cyclisiert wird;
(3) ein Trimethiniumsalz der allgemeinen Formel IV 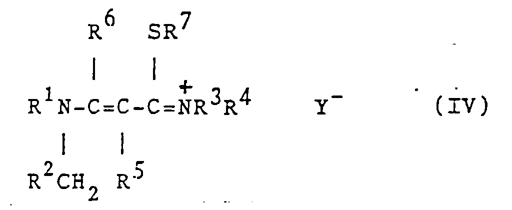 mit R¹ bis R⁶
wie in Anspruch 1,
in der R⁷ eine Alkyl- oder Aralkylgruppe und Y⁻ ein Säurerestanion, wie beispielsweise ein Halogenid, Perchlorat, Alkylsulfat, Sulfonat, Sulfat, Tetrafluor- oder Tetraarylborat oder Picrat, bedeuten, oder ggfs. die zugehörige freie Base eines Trimethiniumsalzes der allgemeinen Formel IV cyclisiert wird, vorzugsweise mit einer Base;
(4) zur Herstellung von Verbindungen der Formel I, in der R¹ und R⁶ Wasserstoff und NR³R⁴ Morpholino bedeuten,
ein Trimethiniumsalz der allgemeinen Formel V 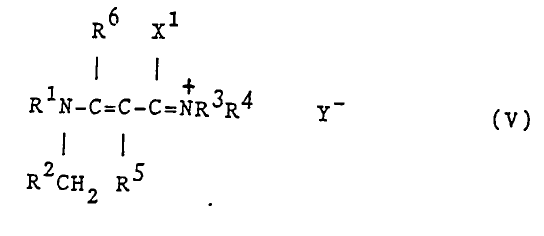 mit R¹ bis R⁶ wie in Anspruch 1,
in der X¹ eine Abgangsgruppe, wie beispielsweise ein Halogen, eine Alkoxy- oder eine Aminogruppe, und Y⁻ ein Säurerestanion, beispielsweise ein Halogenid, Perchlorat, Alkylsulfat, Sulfonat, Sulfat, Tetrafluor- oder Tetraarylborat oder Picrat, bedeuten,
oder ggfs. die zugehörige freie Base eines Trimethiniumsalzes der allgemeinen Formel V cyclisiert wird, vorzugsweise in Gegenwart einer Base;
(5) zur Herstellung von Verbindungen der Formel I, in der R¹ und R⁶ Wasserstoff und NR³R⁴ Morpholino bedeuten,
ein Acrylsäureamidderivat der allgemeinen Formel VI 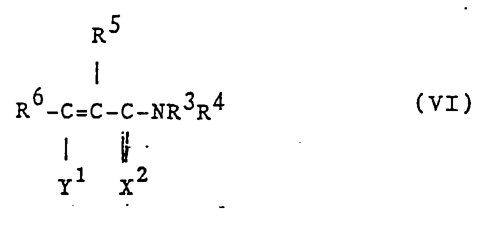 mit R³ bis R⁶ wie in Anspruch 1,
in der X² ein Sauerstoff-, Schwefel- oder ein mono- oder disubstituiertes Stickstoffatom und Y¹ eine Abgangsgruppe, wie beispielsweise eine substituierte oder unsubstituierte Aminogruppe, eine Alkylthio-, eine Alkoxy-, eine Hydroxy-, eine Mercapto- oder eine Acyloxygruppe oder ein Halogen, bedeuten,
mit einem Amin der allgemeinen Formel VII
R²CH₂NHR¹ (VII)
mit R¹ und R² wie in Anspruch 1 umgesetzt wird,
vorzugsweise mit einem Alkylierungsmittel oder einem Oxidationsmittel und vorzugsweise mit einer Base;
(6) zur Herstellung von Verbindungen der Formel I, in der R¹ und R⁶ Wasserstoff und NR³R⁴ Morpholino bedeuten,
ein Iminiumsalz der allgemeinen Formel VIII 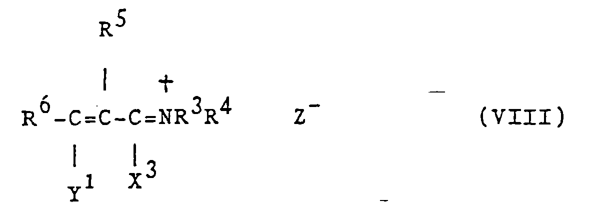 mit R³ bis R⁶ wie in Anspruch 1,
in der X³ und Y¹ gleich oder verschieden sind und eine Abgangsgruppe, wie beispielsweise eine substituierte oder unsubstituierte Aminogruppe, eine Alkylthio-, eine Alkoxy-, eine Hydroxy-, eine Mercapto-, eine Acyloxygruppe oder ein Halogen, und Z⁻ ein Säurerestanion, beispielsweise ein Halogenid, Perchlorat, Alkylsulfat, Sulfonat, Sulfat, Tetrafluor- oder Tetraarylborat oder Picrat, bedeuten,
mit einem Amin der allgemeinen Formel VII wie oben definiert und mit einer Base umgesetzt wird;
(7) zur Herstellung von Verbindungen der Formel I, in der R¹ Methyl, Ethyl, Benzyl oder Ethoxycarbonylmethyl bedeutet,
ein 1-unsubstituiertes 3-Aminopyrrol der allgemeinen Formel IX 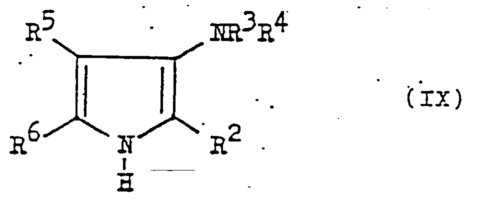 mit R² bis R⁶ wie in Anspruch 1
mit einem Alkylierungsmittel der allgemeinen Formel X
R¹-Y² (X)
mit R¹ wie in Anspruch 1, in der Y² eine Abgangsgruppe, wie beispielsweise ein Halogen, eine Sulfonyloxy- oder eine Diazogruppe, bedeuten, und einer Base umgesetzt wird;
(8) zur Herstellung von Verbindungen der Formel I, in der R² COZ¹ mit Z¹ = Hydroxy, NaO, Methoxy, Ethoxy oder Anilino bedeutet, ein Pyrrolcarbonsäurederivat der allgemeinen Formel XI 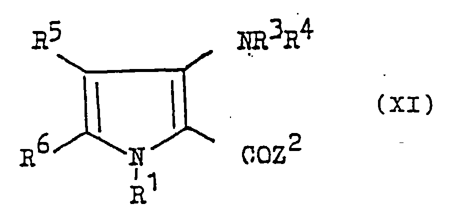 mit R¹ bis R⁶ wie in Anspruch 1, in der Z² verschieden von Z¹ eine Abgangsgruppe, wie beispielsweise eine Hydroxy-, Alkoxy-, Aryloxy-, Acyloxy- oder Alkylthiogruppe oder ein Halogen, bedeutet, mit einem nucleophilen Reagens der allgemeinen Formel XII
Z¹H (XII)
mit Z¹ wie oben
oder der deprotonierten Form eines nucleophilen Reagens der allgemeinen Formel XII
und ggfs. mit einer Säure oder Base umgesetzt wird;
(9) zur Herstellung von Verbindungen der Formel I, in der R¹ und R⁶ Wasserstoff und NR³R⁴ Morpholino bedeuten,
ein Enamin der allgemeinen Formel XIII 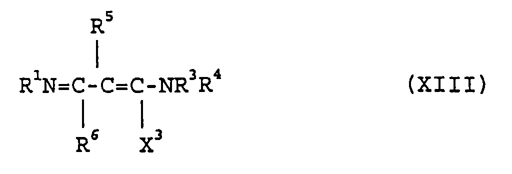 mit R¹ bis R⁶ wie in Anspruch 1,
in der X³ eine Abgangsgruppe, wie beispielsweise eine Alkoxy-, Alkylmercapto- oder substituierte Aminogruppe oder ein Halogen, bedeutet,
oder ein Salz eines Enamins der allgemeinen Formel XIII, wie beispielsweise ein Hydrohalogenid, ein Hydroperchlorat, ein Hydrosulfonat oder ein Hydrotetrafluorborat,
mit einem Alkylierungsmittel der allgemeinen Formel XIV
R²CH₂Z³ (XIV)
mit R² wie oben, in der Z³ eine Abgangsgruppe, wie beispielsweise ein Halogen, ein Sulfonat, ein Alkylsulfat oder ein Trifluoracetat, bedeutet, und einer Base umgesetzt wird;
(10) zur Herstellung von Verbindungen der Formel I, in der R¹ und R⁶ Wasserstoff und NR³R⁴ Morpholino bedeuten,
ein Enamin der allgemeinen Formel XV 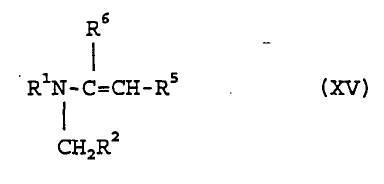 mit R¹, R², R⁵ und R⁶ wie in Anspruch 1
mit einem Iminiumsalz der allgemeinen Formel XVI 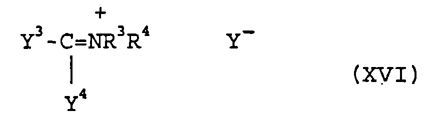 mit R³ und R⁴ wie oben,
in der Y³ und Y⁴ gleiche oder verschiedene Abgangsgruppen, wie beispielsweise Chlor, Aminogruppen, Alkylmercapto- oder Alkoxygruppen, und Y⁻ein Säurerestanion, beispielsweise ein Halogenid, ein Sulfonat, ein Sulfat oder ein Trifluoracetat, darstellen,
und ggfs. mit einer Base umgesetzt wird;
(11) zur Herstellung von Verbindungen der Formel I, in der R²R⁷C=Z⁴ bedeutet, wobei R⁷ Methyl, Methoxy, Ethoxy oder NH-C₆H₅ bedeutet und Z⁴ für ein Sauerstoffatom steht,
ein 2-unsubstituiertes 3-Aminopyrrol der allgemeinen Formel XVII 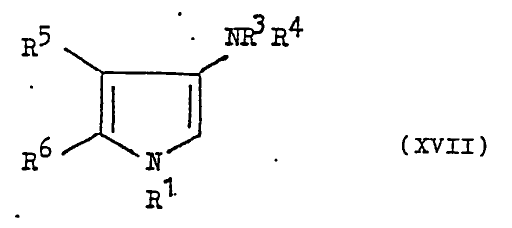 mit R¹ und R³ bis R⁶ wie in Anspruch 1
mit einer Carbonylverbindung der allgemeinen Formel XVIII 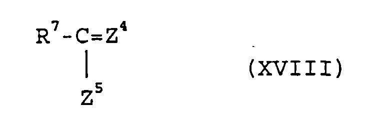 mit R⁷ und Z⁴ wie oben, in der Z⁵ eine Abgangsgruppe, wie beispielsweise ein Halogen oder eine Acyloxygruppe, bedeutet,
oder, sofern bei den Verbindungen der Formel I mit R² = R⁷C=Z⁴ R⁷ NHC₆H₅ bedeutet,
auch mit einem Heterocumulen der allgemeinen Formel XIX
R⁵N=C=Z⁴ (XIX)
mit Z⁴ und R⁵ wie oben
und ggfs. mit einer Lewis- oder Protonsäure umgesetzt wird;
(12) zur Herstellung von Verbindungen der Formel I, in der R¹ und R⁶ Wasserstoff und NR³R⁴ Morpholino bedeuten,
ein 3-substituiertes Pyrrol der allgemeinen Formel XX 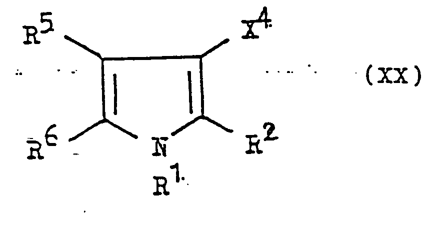 mit R¹, R², R⁵ und R⁶ wie in Anspruch 1,
in der X⁴ eine Abgangsgruppe, wie ein Halogen, eine Hydroxy- oder Alkoxygruppe, eine Alkylthiogruppe oder eine Diazoniumgruppe, bedeutet,
mit einem Amin der allgemeinen Formel XXI
HNR³R⁴ (XXI)
mit R³ und R⁴ wie in Anspruch 1
umgesetzt wird;
(13) zur Herstellung von Verbindungen der Formel I, in der R² COOR⁷ bedeutet, wobei R⁷ Methyl oder Ethyl darstellt,
eine 3-Aminopyrrolcarbonsäure der allgemeinen Formel XXII 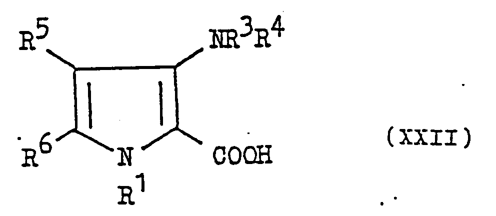 mit R¹, R³, R⁴, R⁵ und R⁶ wie in Anspruch 1
oder ein Salz einer 3-Aminopyrrolcarbonsäure der allgemeinen Formel XXII, beispielsweise ein Natrium-, Kalium- oder Ammoniumsalz, mit einem Alkylierungsmittel der allgemeinen Formel XXIII
R⁷X⁵ (XXIII)
mit R⁷ wie oben, in der X⁵ eine Abgangsgruppe, beispielsweise ein Halogen oder eine Sulfonylgruppe, bedeutet,
und ggfs. mit einer Base umgesetzt wird.

3. Verfahren nach Anspruch 2, Varianten 1 bis 9, dadurch gekennzeichnet, daß als Base ein Amin, ein Alkali- oder Erdalkalihydroxid oder -hydrid, ein Alkalicarbonat oder ein Metallamid verwendet wird.

4. Verfahren nach Anspruch 2, Varianten 1, 2 und 5, dadurch gekennzeichnet, daß als Alkylierungsmittel ein Alkyl- oder Aralkylhalogenid, ein Alkylsulfonat, ein Dialkylsulfat, ein Trialkyloxoniumsalz oder Diazomethan verwendet werden.

5. Verfahren nach Anspruch 2, Varianten 1 und 5, dadurch gekennzeichnet, daß als Oxidationsmittel Wasserstoffperoxid, eine Persäure oder ein Persulfat verwendet werden.

6. Pharmazeutische Zusammensetzungen, gekennzeichnet durch ein oder mehrere 3-Aminopyrrole als Wirkstoffe neben einem oder mehreren physiologisch verträglichen Hilfs- und/oder Tragerstoffen und ggfs. einem Verdünnungsmittel, wobei die 3-Aminopyrrole unter den Verbindungen der Formel I nach Anspruch 1 sowie weiteren Verbindungen der Formel I wie in Anspruch 1 mit den nachstehenden Kombinationen der Substituenten R¹ bis R⁶ ausgewählt sind:
| R¹ | R² | R³ | R⁴ | R⁵ | R⁶ |
|---|---|---|---|---|---|
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |

7. Pharmazeutische Zusammensetzungen nach Anspruch 6, insbesondere mit ZNS-Wirksamkeit und vorzugsweise antikonvulsiver und/oder analgetischer Wirkung, gekennzeichnet durch Aminopyrrole der Formel I wie in Anspruch 6 definiert in einer Menge von insgesamt 0,5 bis 20 mg/kg pro Einzeldosis.

8. Verwendung der 3-Aminopyrrole der Formel I nach Anspruch 1 sowie der in Anspruch 6 aufgeführten weiteren Verbindungen der Formel I als oder zur Herstellung von pharmazeutischen Mitteln zur Behandlung von Erkrankungen des Zentralnervensystems, insbesondere von Epilepsien verschiedener Formen, nichtepileptischen Anfällen, Migräne und Schmerzen verschiedener Genese.

9. Verfahren zur Herstellung der pharmazeutischen Zusammensetzungen nach Anspruch 6 oder 7, dadurch gekennzeichnet, daß der oder die Wirkstoffe unter Verwendung pharmazeutisch geeigneter Hilfs- und/oder Trägerstoffe und/oder Verdünnungsmittel in an sich bekannter Weise in eine galenisch geeignete Form gebracht werden.

## Patentansprüche (Patentansprüche für folgende(n) Vertragsstaat(en): ES)

1. Verfahren zur Herstellung von
3-Aminopyrrolen der Formel I, 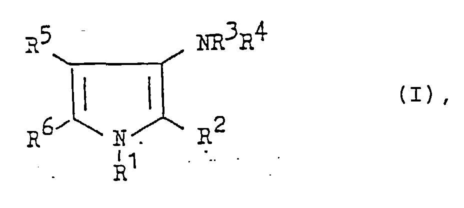 in der die Substituenten R¹ bis R⁶ die den nachstehenden Kombinationen entsprechenden Bedeutungen besitzen:
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
dadurch gekennzeichnet, daß
(1) ein Aminoacrylsäurederivat der allgemeinen Formel II 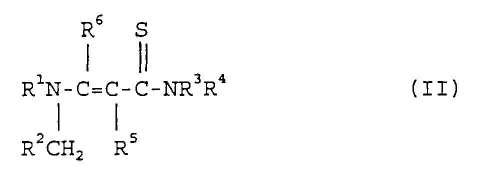 mit R¹ bis R⁶ wie in Anspruch 1
cyclisiert wird,
vorzugsweise in Gegenwart eines Alkylierungsmittels oder eines Oxidationsmittels und vorzugsweise in Gegenwart einer Base;
(2) zur Herstellung von Verbindungen der Formel I, in der R¹ und R⁶ Wasserstoff und NR³R⁴ Morpholino bedeuten,
ein Aminoacrylsäurederivat der allgemeinen Formel III, 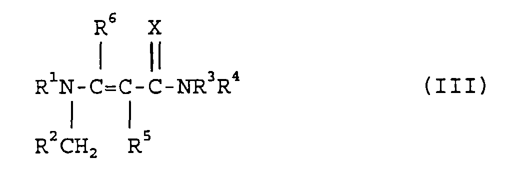 mit R¹ bis R⁶ wie in Anspruch 1,
in der X Sauerstoff oder ein substituiertes Stickstoffatom bedeutet,
gegebenenfalls in Gegenwart eines Alkylierungsmittels oder eines Dehydratisierungsmittels und vorzugsweise in Gegenwart einer Base,
cyclisiert wird;
(3) ein Trimethiniumsalz der allgemeinen Formel IV 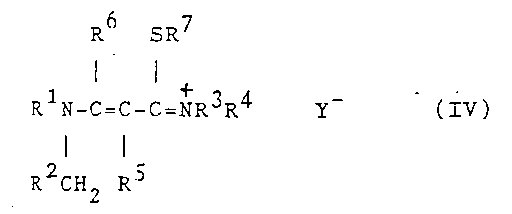 mit R¹ bis R⁶
wie in Anspruch 1,
in der R⁷ eine Alkyl- oder Aralkylgruppe und Y⁻ ein Säurerestanion, wie beispielsweise ein Halogenid, Perchlorat, Alkylsulfat, Sulfonat, Sulfat, Tetrafluor- oder Tetraarylborat oder Picrat, bedeuten, oder ggfs. die zugehörige freie Base eines Trimethiniumsalzes der allgemeinen Formel IV cyclisiert wird, vorzugsweise mit einer Base;
(4) zur Herstellung von Verbindungen der Formel I, in der R¹ und R⁶ Wasserstoff und NR³R⁴ Morpholino bedeuten,
ein Trimethiniumsalz der allgemeinen Formel V 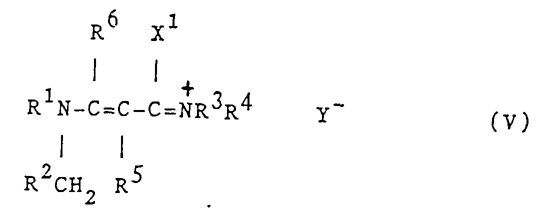 mit R¹ bis R⁶ wie in Anspruch 1,
in der X¹ eine Abgangsgruppe, wie beispielsweise ein Halogen, eine Alkoxy- oder eine Aminogruppe, und Y⁻ ein Säurerestanion, beispielsweise ein Halogenid, Perchlorat, Alkylsulfat, Sulfonat, Sulfat, Tetrafluor- oder Tetraarylborat oder Picrat, bedeuten,
oder ggfs. die zugehörige freie Base eines Trimethiniumsalzes der allgemeinen Formel V cyclisiert wird, vorzugsweise in Gegenwart einer Base;
(5) zur Herstellung von Verbindungen der Formel I, in der R¹ und R⁶ Wasserstoff und NR³R⁴ Morpholino bedeuten,
ein Acrylsäureamidderivat der allgemeinen Formel VI 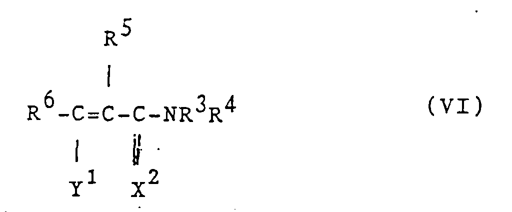 mit R³ bis R⁶ wie in Anspruch 1,
in der X² ein Sauerstoff-, Schwefel- oder ein mono- oder disubstituiertes Stickstoffatom und Y¹ eine Abgangsgruppe, wie beispielsweise eine substituierte oder unsubstituierte Aminogruppe, eine Alkylthio-, eine Alkoxy-, eine Hydroxy-, eine Mercapto- oder eine Acyloxygruppe oder ein Halogen, bedeuten,
mit einem Amin der allgemeinen Formel VII
R²CH₂NHR¹ (VII)
mit R¹ und R² wie in Anspruch 1 umgesetzt wird,
vorzugsweise mit einem Alkylierungsmittel oder einem Oxidationsmittel und vorzugsweise mit einer Base;
(6) zur Herstellung von Verbindungen der Formel I, in der R¹ und R⁶ Wasserstoff und NR³R⁴ Morpholino bedeuten,
ein Iminiumsalz der allgemeinen Formel VIII 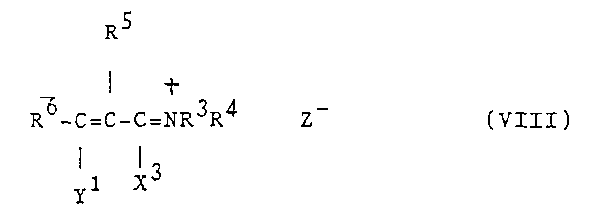 mit R³ bis R⁶ wie in Anspruch 1,
in der X³ und Y¹ gleich oder verschieden sind und eine Abgangsgruppe, wie beispielsweise eine substituierte oder unsubstituierte Aminogruppe, eine Alkylthio-, eine Alkoxy-, eine Hydroxy-, eine Mercapto-, eine Acyloxygruppe oder ein Halogen, und Z⁻ ein Säurerestanion, beispielsweise ein Halogenid, Perchlorat, Alkylsulfat, Sulfonat, Sulfat, Tetrafluor- oder Tetraarylborat oder Picrat, bedeuten,
mit einem Amin der allgemeinen Formel VII wie oben definiert und mit einer Base umgesetzt wird;
(7) zur Herstellung von Verbindungen der Formel I, in der R¹ Methyl, Ethyl, Benzyl oder Ethoxycarbonylmethyl bedeutet,
ein 1-unsubstituiertes 3-Aminopyrrol der allgemeinen Formel IX 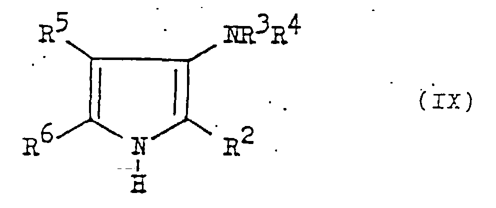 mit R² bis R⁶ wie in Anspruch 1
mit einem Alkylierungsmittel der allgemeinen Formel X
R¹-Y² (X)
mit R¹ wie in Anspruch 1, in der Y² eine Abgangsgruppe, wie beispielsweise ein Halogen, eine Sulfonyloxy- oder eine Diazogruppe, bedeuten, und einer Base umgesetzt wird;
(8) zur Herstellung von Verbindungen der Formel I, in der R² COZ¹ mit Z¹ = Hydroxy, NaO, Methoxy, Ethoxy oder Anilino bedeutet,
ein Pyrrolcarbonsäurederivat der allgemeinen Formel XI 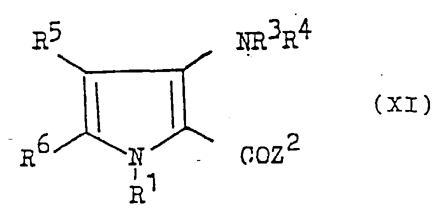 mit R¹ bis R⁶ wie in Anspruch 1, in der Z² verschieden von Z¹ eine Angangsgruppe, wie beispielsweise eine Hydroxy-, Alkoxy-, Aryloxy-, Acyloxy- oder Alkylthiogruppe oder ein Halogen, bedeutet, mit einem nucleophilen Reagens der allgemeinen Formel XII
Z¹H (XII)
mit Z¹ wie oben
oder der deprotonierten Form eines nucleophilen Reagens der allgemeinen Formel XII
und ggfs. mit einer Säure oder Base umgesetzt wird;
(9) zur Herstellung von Verbindungen der Formel I, in der R¹ und R⁶ Wasserstoff und NR³R⁴ Morpholino bedeuten,
ein Enamin der allgemeinen Formel XIII 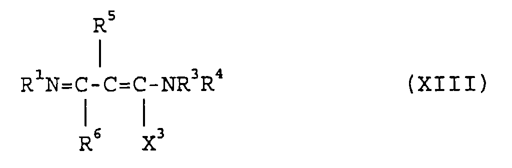 mit R¹ bis R⁶ wie in Anspruch 1,
in der X³ eine Angangsgruppe, wie beispielsweise eine Alkoxy-, Alkylmercapto- oder substituierte Aminogruppe oder ein Halogen, bedeutet,
oder ein Salz eines Enamins der allgemeinen Formel XIII, wie beispielsweise ein Hydrohalogenid, ein Hydroperchlorat, ein Hydrosulfonat oder ein Hydrotetrafluorborat,
mit einem Alkylierungsmittel der allgemeinen Formel XIV
R²CH₂Z³ (XIV)
mit R² wie oben, in der Z³ eine Abgangsgruppe, wie beispielsweise ein Halogen, ein Sulfonat, ein Alkylsulfat oder ein Trifluoracetat, bedeutet, und einer Base umgesetzt wird;
(10) zur Herstellung von Verbindungen der Formel I, in der R¹ und R⁶ Wasserstoff und NR³R⁴ Morpholino bedeuten,
ein Enamin der allgemeinen Formel XV 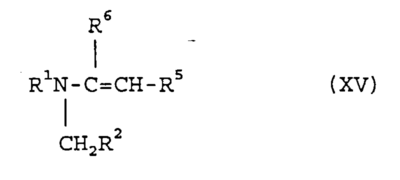 mit R¹, R², R⁵ und R⁶ wie in Anspruch 1
mit einem Iminiumsalz der allgemeinen Formel XVI 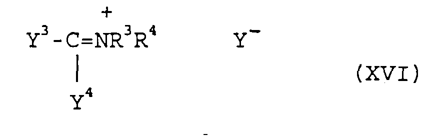 mit R³ und R⁴ wie oben,
in der Y³ und Y⁴ gleiche oder verschiedene Abgangsgruppen, wie beispielsweise Chlor, Aminogruppen, Alkylmercapto- oder Alkoxygruppen, und Y⁻ ein Säurerestanion, beispielsweise ein Halogenid, ein Sulfonat, ein Sulfat oder ein Trifluoracetat, darstellen,
und ggfs. mit einer Base umgesetzt wird;
(11) zur Herstellung von Verbindungen der Formel I, in der R²R⁷C=Z⁴ bedeutet, wobei R⁷ Methyl, Methoxy, Ethoxy oder NH-C₆H₅ bedeutet und Z⁴ für ein Sauerstoffatom steht,
ein 2-unsubstituiertes 3-Aminopyrrol der allgemeinen Formel XVII 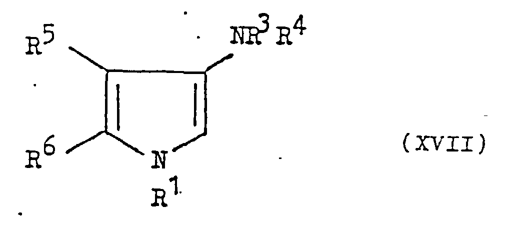 mit R¹ und R³ bis R⁶ wie in Anspruch 1
mit einer Carbonylverbindung der allgemeinen Formel XVIII 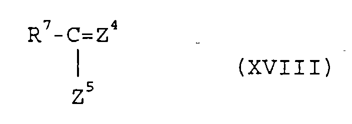 mit R⁷ und Z⁴ wie oben, in der Z⁵ eine Abgangsgruppe, wie beispielsweise ein Halogen oder eine Acyloxygruppe, bedeutet,
oder, sofern bei den Verbindungen der Formel I mit R² = R⁷C=Z⁴ R⁷ NHC₆H₅ bedeutet,
auch mit einem Heterocumulen der allgemeinen Formel XIX
R⁵N=C=Z⁴ (XIX)
mit Z⁴ und R⁵ wie oben
und ggfs. mit einer Lewis- oder Protonsäure umgesetzt wird;
(12) zur Herstellung von Verbindungen der Formel I, in der R¹ und R⁶ Wasserstoff und NR³R⁴ Morpholino bedeuten,
ein 3-substituiertes Pyrrol der allgemeinen Formel XX 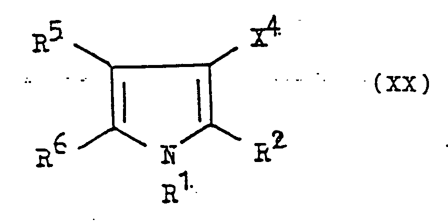 mit R¹, R², R⁵ und R⁶ wie in Anspruch 1,
in der X⁴ eine Abgangsgruppe, wie ein Halogen, eine Hydroxy- oder Alkoxygruppe, eine Alkylthiogruppe oder eine Diazoniumgruppe, bedeutet,
mit einem Amin der allgemeinen Formel XXI
HNR³R⁴ (XXI)
mit R³ und R⁴ wie in Anspruch 1
umgesetzt wird;
(13) zur Herstellung von Verbindungen der Formel I, in der R² COOR⁷ bedeutet, wobei R⁷ Methyl oder Ethyl darstellt,
eine 3-Aminopyrrolcarbonsäure der allgemeinen Formel XXII 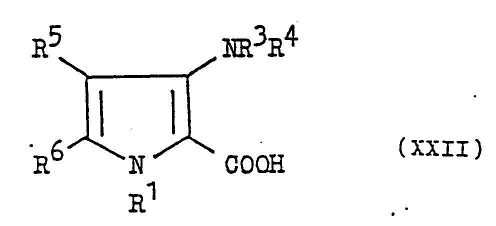 mit R¹, R³, R⁴, R⁵ und R⁶ wie in Anspruch 1
oder ein Salz einer 3-Aminopyrrolcarbonsäure der allgemeinen Formel XXII, beispielsweise ein Natrium-, Kalium- oder Ammoniumsalz, mit einem Alkylierungsmittel der allgemeinen Formel XXIII
R⁷X⁵ (XXIII)
mit R⁷ wie oben, in der X⁵ eine Abgangsgruppe, beispielsweise ein Halogen oder eine Sulfonylgruppe, bedeutet,
und ggfs. mit einer Base umgesetzt wird.

2. Verfahren nach Anspruch 1, Varianten 1 bis 9, dadurch gekennzeichnet, daß als Base ein Amin, ein Alkali- oder Erdalkalihydroxid oder -hydrid, ein Alkalicarbonat oder ein Metallamid verwendet wird.

3. Verfahren nach Anspruch 1, Varianten 1, 2 und 5, dadurch gekennzeichnet, daß als Alkylierungsmittel ein Alkyl- oder Aralkylhalogenid, ein Alkylsulfonat, ein Dialkylsulfat, ein Trialkyloxoniumsalz oder Diazomethan verwendet werden.

4. Verfahren nach Anspruch 1, Varianten 1 und 5, dadurch gekennzeichnet, daß als Oxidationsmittel Wasserstoffperoxid, eine Persäure oder ein Persulfat verwendet werden.

5. Verwendung von 3-Aminopyrrolen der Formel I 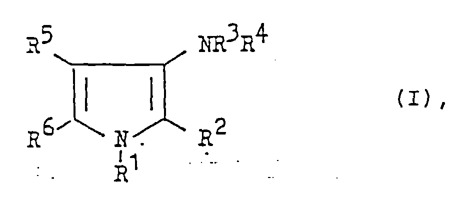 in der die Substituenten R¹ bis R⁶ die den nachstehenden Kombinationen entsprechenden Bedeutungen besitzen:
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |
als pharmazeutische Mittel oder zur Herstellung von pharmazeutischen Mitteln, insbesondere mit ZNS-Wirksamkeit und vorzugsweise antikonvulsiver und/oder analgetischer Wirkung,

6. Verwendung von 3-Aminopyrrolen der Formel I nach Anspruch 5 zur Herstellung von pharmazeutischen Mitteln zur Behandlung von Erkrankungen des Zentralnervensystems, insbesondere von Epilepsien verschiedener Formen, nichtepileptischen Anfällen, Migräne und Schmerzen verschiedener Genese.

7. Verfahren zur Herstellung von pharmazeutischen Zusammensetzungen, dadurch gekennzeichnet, daß ein oder mehrere 3-Aminopyrrole der Formel I nach Anspruch 5 unter Verwendung pharmazeutisch geeigneter Hilfs- und/oder Trägerstoffe und/oder Verdünnungsmittel in an sich bekannter Weise in eine galenisch geeignete Form gebracht werden.

## Claims (Claims for the following Contracting State(s): BE, CH, LI, DE, FR, GB, IT, NL, SE)

1. 3-Aminopyrroles of the formula I 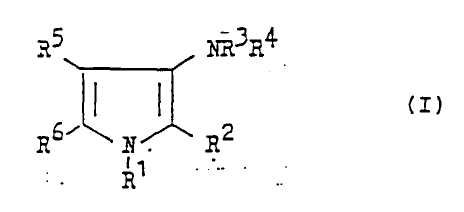 in which the substituents R¹ to R⁶ have the meanings corresponding to the following combinations:
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |

2. Process for the preparation of the 3-aminopyrroles of the formula I according to claim 1,
characterized in that
(1) an aminoacrylic acid derivative of the general formula II 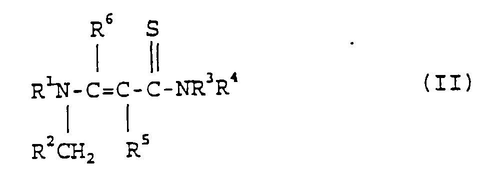 where R¹ to R⁶ are as in claim 1
is cyclized,
preferably in the presence of an alkylating agent or an oxidizing agent and preferably in the presence of a base;
(2) to prepare compounds of the formula I in which R¹ and R⁶ denote hydrogen and NR³R⁴ denotes morpholino, an aminoacrylic acid derivative of the general formula III, 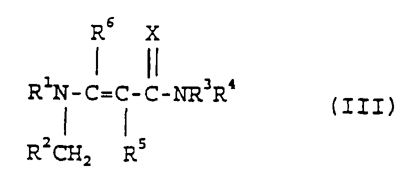 where R¹ to R⁶ are as in claim 1,
in which X denotes oxygen or a substituted nitrogen atom,
is cyclized,
if appropriate in the presence of an alkylating agent or a dehydrating agent and preferably in the presence of a base;
(3) a trimethinium salt of the general formula IV 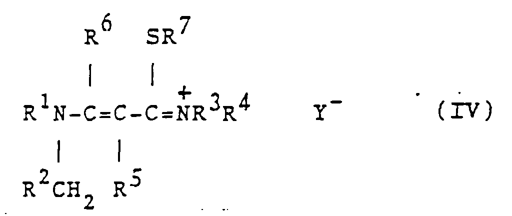 where R¹ to R⁵
are as in claim 1,
in which R⁷ denotes an alkyl or aralkyl group and Y⁻denotes an acid radical anion, such as, for example, a halide, perchlorate, alkylsulphate, sulphonate, sulphate, tetrafluoro- or tetraarylborate or picrate,
or, if appropriate, the associated free base of a trimethinium salt of the general formula IV, is cyclized, preferably with a base;
(4) to prepare compounds of the formula I in which R¹ and R⁶ denote hydrogen and NR³R⁴ denotes morpholino, a trimethinium salt of the general formula V 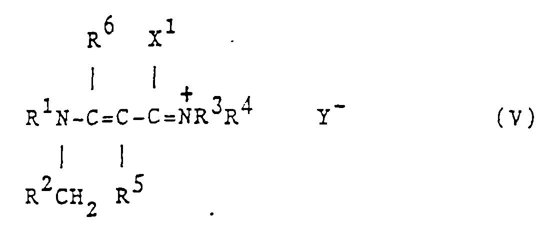 where R¹ to R⁶ are as in claim 1,
in which X¹ denotes a leaving group, such as, for example, a halogen or an alkoxy or an amino group, and Y⁻ denotes an acid radical anion, for example a halide, perchlorate, alkylsulphate, sulphonate, sulphate, tetrafluoro- or tetraarylborate or picrate,
or, if appropriate, the associated free base of a trimethinium salt of the general formula V, is cyclized, preferably in the presence of a base;
(5) to prepare compounds of the formula I in which R¹ and R⁶ denote hydrogen and NR³R⁴ denotes morpholino, an acrylic acid amide derivative of the general formula VI 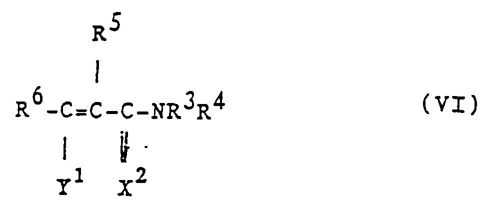 where R³ to R⁶ are as in claim 1,
in which X² denotes an oxygen, sulphur or a mono- or disubstituted nitrogen atom and Y¹ denotes a leaving group, such as, for example, a substituted or unsubstituted amino group, an alkylthio, an alkoxy, a hydroxyl, a mercapto or an acyloxy group or a halogen,
is reacted with an amine of the general formula VII
R²CH₂NHR¹ (VII)
where R¹ and R² are as in claim 1,
preferably with an alkylating agent or an oxidizing agent and preferably with a base;
(6) to prepare compounds of the formula I in which R¹ and R⁶ denote hydrogen and NR³R⁴ denotes morpholino, an iminium salt of the general formula VIII 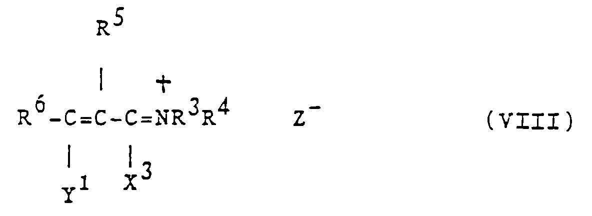 where R³ to R⁶ are as in claim 1,
in which X³ and Y¹ are identical or different and denote a leaving group, such as, for example, a substituted or unsubstituted amino group, an alkylthio, an alkoxy, a hydroxyl, a mercapto or an acyloxy group or a halogen, and Z⁻ denotes an acid radical anion, for example a halide, perchlorate, alkylsulphate, sulphonate, sulphate, tetrafluoro- or tetraarylborate or picrate,
is reacted with an amine of the general formula VII as defined above and with a base;
(7) to prepare compounds of the formula I in which R¹ denotes methyl, ethyl, benzyl or ethoxycarbonylmethyl,
a 1-unsubstituted 3-aminopyrrole of the general formula IX 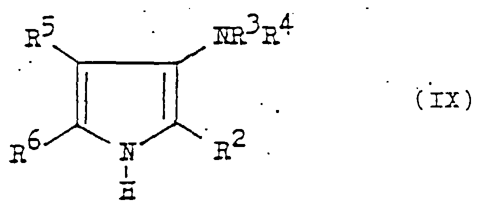 where R² to R⁶ are as in claim 1
is reacted with an alkylating agent of the general formula X
R¹-Y² (X)
where R¹ is as in claim 1, in which Y² denotes a leaving group, such as, for example, a halogen or a sulphonyloxy or a diazo group, and a base;
(8) to prepare compounds of the formula I in which R² denotes COZ¹, where Z¹ = hydroxyl, NaO, methoxy, ethoxy or anilino,
a pyrrolecarboxylic acid derivative of the general formula XI 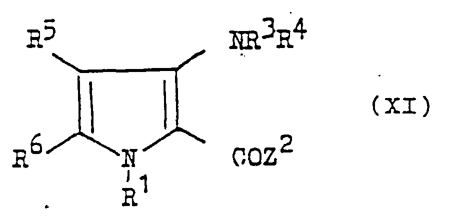 where R¹ to R⁶ are as in claim 1, in which Z² denotes a leaving group which differs from Z¹, such as, for example, a hydroxyl, alkoxy, aryloxy, acyloxy or alkylthio group or a halogen, is reacted with a nucleophilic reagent of the general formula XII
Z¹H (XII)
where Z¹ is as above
or the deprotonated form of a nucleophilic reagent of the general formula XII
and if appropriate with an acid or base;
(9) to prepare compounds of the formula I in which R¹ and R⁶ denote hydrogen and NR³R⁴ denotes morpholino,
an enamine of the general formula XIII 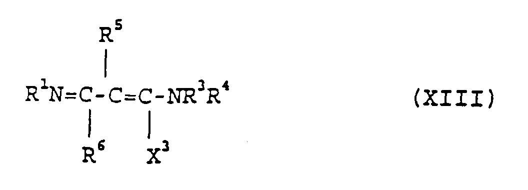 where R¹ to R⁶ are as in claim 1,
in which X³ denotes a leaving group, such as, for example, an alkoxy, alkylmercapto or substituted amino group or a halogen,
or a salt of an enamine of the general formula XIII, such as, for example, a hydrohalide, a hydroperchlorate, a hydrosulphonate or a hydrotetrafluoroborate,
is reacted with an alkylating agent of the general formula XIV
R²CH₂Z³ (XIV)
where R² is as above, in which Z³ denotes a leaving group, such as, for example, a halogen, a sulphonate, an alkylsulphate or a trifluoroacetate, and a base;
(10) to prepare compounds of the formula I in which R¹ and R⁶ denote hydrogen and NR³R⁴ denotes morpholino, an enamine of the general formula XV 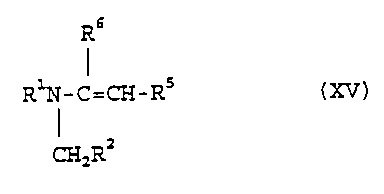 where R¹, R², R⁵ and R⁶ are as in claim 1 is reacted with an iminium salt of the general formula XVI 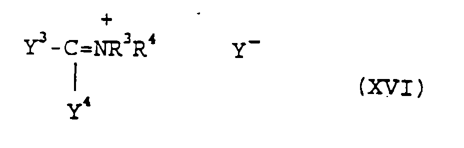 where R³ and R⁴ are as above,
in which Y³ and Y⁴ represent identical or different leaving groups, such as, for example, chlorine, amino groups or alkylmercapto or alkoxy groups, and Y⁻ represents an acid radical anion, for example a halide, a sulphonate, a sulphate or a trifluoroacetate,
and if appropriate with a base;
(11) to prepare compounds of the formula I in which R² denotes R⁷C=Z⁴, wherein R⁷ denotes methyl, methoxy, ethoxy or NH-C₆H₅ and Z⁴ represents an oxygen atom,
a 2-unsubstituted 3-aminopyrrole of the general formula XVII 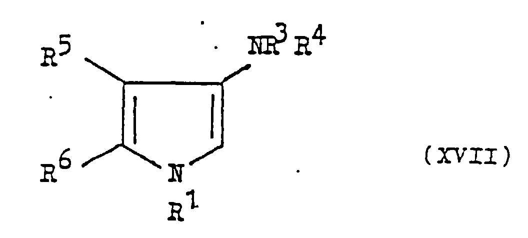 where R¹ and R³ to R⁶ are as in claim 1
is reacted with a carbonyl compound of the general formula XVIII 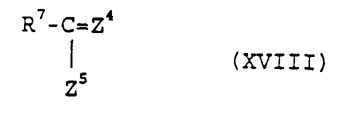 where R⁷ and Z⁴ are as above, in which Z⁵ denotes a leaving group, such as, for example, a halogen or an acyloxy group,
or if, in the compounds of the formula I where R² = R⁷C=Z⁴, R⁷ denotes NHC₆H₅,
also with a heterocumulene of the general formula XIX
R⁵N=C=Z⁴ (XIX)
where Z⁴ and R⁵ are as above
and if appropriate with a Lewis or proton acid;
(12) to prepare compounds of the formula I in which R¹ and R⁶ denote hydrogen and NR³R⁴ denotes morpholino,
a 3-substituted pyrrole of the general formula XX 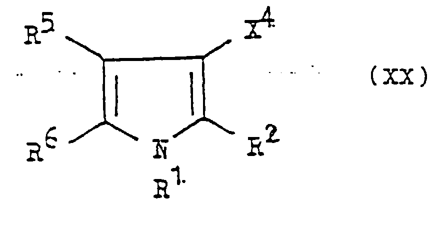 where R¹, R², R⁵ and R⁶ are as in claim 1,
in which X⁴ denotes a leaving group, such as a halogen, a hydroxyl or alkoxy group, an alkylthio group or a diazonium group,
is reacted with an amine of the general formula XXI
HNR³R⁴ (XXI)
where R³ and R⁴ are as in claim 1;
(13) to prepare compounds of the formula I in which R² denotes COOR⁷, wherein R⁷ represents methyl or ethyl,
a 3-aminopyrrolecarboxylic acid of the general formula XXII 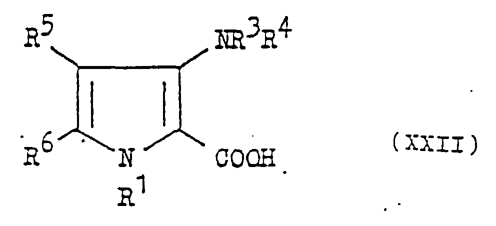 where R¹, R³, R⁴, R⁵ and R⁶ are as in claim 1,
or a salt of a 3-aminopyrrolecarboxylic acid of the general formula XXII, for example a sodium, potassium or ammonium salt, is reacted with an alkylating agent of the general formula XXIII
R⁷X⁵ (XXIII)
where R⁷ is as above, in which X⁵ denotes a leaving group, for example a halogen or a sulphonyl group,
and if appropriate with a base.

3. Process according to claim 2, variants 1 to 9, characterized in that an amine, an alkali metal or alkaline earth metal hydroxide or hydride, an alkali metal carbonate or a metal amide is used as the base.

4. Process according to claim 2, variants 1, 2 and 5, characterized in that an alkyl or aralkyl halide, an alkylsulphonate, a dialkyl sulphate, a trialkyloxonium salt or diazomethane is used as the alkylating agent.

5. Process according to claim 2, variants 1 and 5, characterized in that hydrogen peroxide, a peracid or a persulphate is used as the oxidizing agent.

6. Pharmaceutical compositions, characterized by one or more 3-aminopyrroles as active compounds, in addition to one or more physiologically tolerated auxiliaries and/or carriers, and if appropriate a diluent, the 3-aminopyrroles being chosen from the compounds of the formula I according to claim 1 and further compounds of the formula I as in claim 1 with the following combinations of the substituents R¹ to R⁶:
| R¹ | R² | R³ | R⁴ | R⁵ | R⁶ |
|---|---|---|---|---|---|
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₅H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |

7. Pharmaceutical compositions according to claim 6, in particular having a CNS activity and preferably an anticonvulsive and/or analgesic action, characterized by aminopyrroles of the formula I as defined in claim 6 in an amount of 0.5 to 20 mg/kg in total per individual dose.

8. Use of the 3-aminopyrroles of the formula I according to claim 1 and of the other compounds of the formula I listed in claim 6 as or for the preparation of pharmaceutical agents for treatment of diseases of the central nervous system, in particular epilepsies in various forms, non-epileptic fits, migraines and pain of various origins.

9. Process for the preparation of the pharmaceutical compositions according to claim 6 or 7, characterized in that the active compound or compounds are brought into a galenically suitable form in a manner known per se using pharmaceutically suitable auxiliaries and/or carriers and/or diluents.

## Claims (Claims for the following Contracting State(s): ES)

1. Process for the preparation of 3-aminopyrroles of the formula I 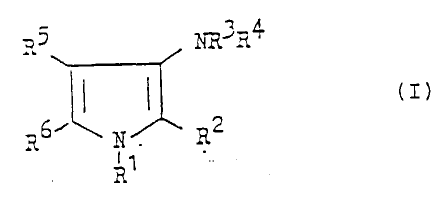 in which the substituents R¹ to R⁶ have the meanings corresponding to the following combinations:
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
characterized in that
(1) an aminoacrylic acid derivative of the general formula II 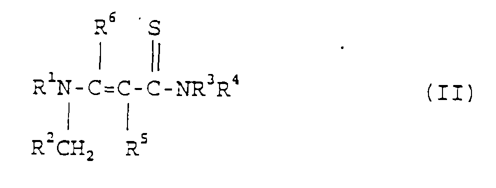 where R¹ to R⁶ are as in claim 1
is cyclized,
preferably in the presence of an alkylating agent or an oxidizing agent and preferably in the presence of a base;
(2) to prepare compounds of the formula I in which R¹ and R⁶ denote hydrogen and NR³R⁴ denotes morpholino, an aminoacrylic acid derivative of the general formula III, 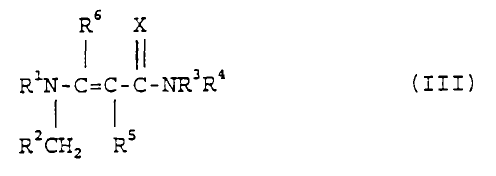 where R¹ to R⁶ are as in claim 1,
in which X denotes oxygen or a substituted nitrogen atom,
is cyclized,
if appropriate in the presence of an alkylating agent or a dehydrating agent and preferably in the presence of a base;
(3) a trimethinium salt of the general formula IV 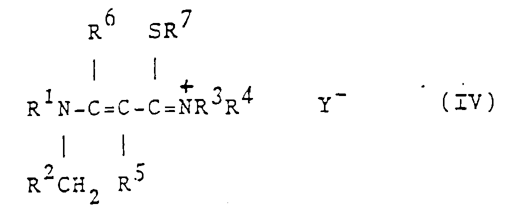 where R¹ to R⁶
are as in claim 1,
in which R⁷ denotes an alkyl or aralkyl group and Y⁻denotes an acid radical anion, such as, for example, a halide, perchlorate, alkylsulphate, sulphonate, sulphate, tetrafluoro- or tetraarylborate or picrate,
or, if appropriate, the associated free base of a trimethinium salt of the general formula IV, is cyclized, preferably with a base;
(4) to prepare compounds of the formula I in which R¹ and R⁶ denote hydrogen and NR³R⁴ denotes morpholino, a trimethinium salt of the general formula V 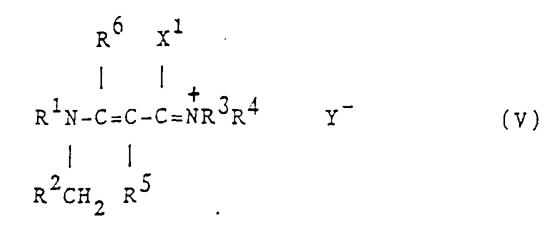 where R¹ to R⁶ are as in claim 1,
in which X¹ denotes a leaving group, such as, for example, a halogen or an alkoxy or an amino group, and Y⁻ denotes an acid radical anion, for example a halide, perchlorate, alkylsulphate, sulphonate, sulphate, tetrafluoro- or tetraarylborate or picrate,
or, if appropriate, the associated free base of a trimethinium salt of the general formula V, is cyclized, preferably in the presence of a base;
(5) to prepare compounds of the formula I in which R¹ and R⁶ denote hydrogen and NR³R⁴ denotes morpholino, an acrylic acid amide derivative of the general formula VI 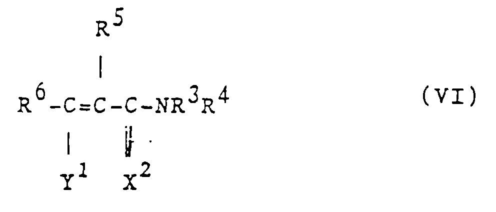 where R³ to R⁶ are as in claim 1,
in which X² denotes an oxygen, sulphur or a mono- or disubstituted nitrogen atom and Y¹ denotes a leaving group, such as, for example, a substituted or unsubstituted amino group, an alkylthio, an alkoxy, a hydroxyl, a mercapto or an acyloxy group or a halogen,
is reacted with an amine of the general formula VII
R²CH₂NHR¹ (VII)
where R¹ and R² are as in claim 1,
preferably with an alkylating agent or an oxidizing agent and preferably with a base;
(6) to prepare compounds of the formula I in which R¹ and R⁶ denote hydrogen and NR³R⁴ denotes morpholino, an iminium salt of the general formula VIII 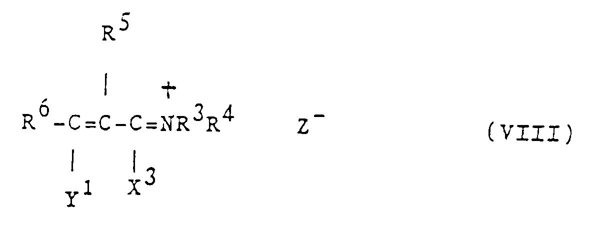 where R³ to R⁶ are as in claim 1,
in which X³ and Y¹ are identical or different and denote a leaving group, such as, for example, a substituted or unsubstituted amino group, an alkylthio, an alkoxy, a hydroxyl, a mercapto or an acyloxy group or a halogen, and Z⁻ denotes an acid radical anion, for example a halide, perchlorate, alkylsulphate, sulphonate, sulphate, tetrafluoro- or tetraarylborate or picrate,
is reacted with an amine of the general formula VII as defined above and with a base;
(7) to prepare compounds of the formula I in which R¹ denotes methyl, ethyl, benzyl or ethoxycarbonylmethyl,
a 1-unsubstituted 3-aminopyrrole of the general formula IX 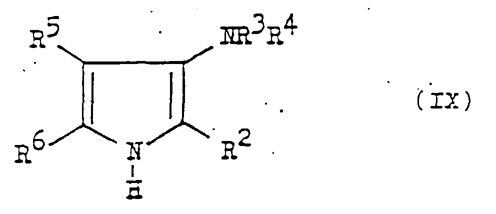 where R² to R⁶ are as in claim 1
is reacted with an alkylating agent of the general formula X
R¹-Y² (X)
where R¹ is as in claim 1, in which Y² denotes a leaving group, such as, for example, a halogen or a sulphonyloxy or a diazo group, and a base;
(8) to prepare compounds of the formula I in which R² denotes COZ¹, where Z¹ = hydroxyl, NaO, methoxy, ethoxy or anilino,
a pyrrolecarboxylic acid derivative of the general formula XI 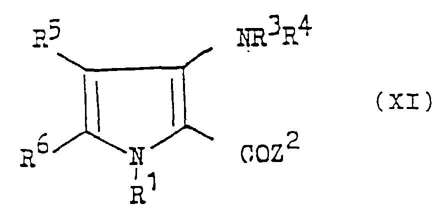 where R¹ to R⁶ are as in claim 1, in which Z² denotes a leaving group which differs from Z¹, such as, for example, a hydroxyl, alkoxy, aryloxy, acyloxy or alkylthio group or a halogen, is reacted with a nucleophilic reagent of the general formula XII
Z¹H (XII)
where Z¹ is as above
or the deprotonated form of a nucleophilic reagent of the general formula XII
and if appropriate with an acid or base;
(9) to prepare compounds of the formula I in which R¹ and R⁶ denote hydrogen and NR³R⁴ denotes morpholino, an enamine of the general formula XIII 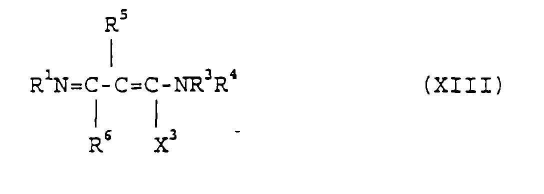 where R¹ to R⁶ are as in claim 1,
in which X³ denotes a leaving group, such as, for example, an alkoxy, alkylmercapto or substituted amino group or a halogen,
or a salt of an enamine of the general formula XIII, such as, for example, a hydrohalide, a hydroperchlorate, a hydrosulphonate or a hydrotetrafluoroborate,
is reacted with an alkylating agent of the general formula XIV
R²CH₂Z³ (XIV)
where R² is as above, in which Z³ denotes a leaving group, such as, for example, a halogen, a sulphonate, an alkylsulphate or a trifluoroacetate, and a base;
(10) to prepare compounds of the formula I in which R¹ and R⁶ denote hydrogen and NR³R⁴ denotes morpholino,
an enamine of the general formula XV 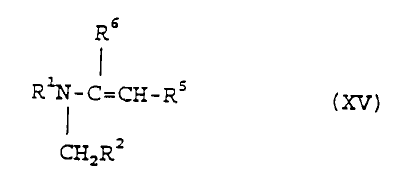 where R¹, R², R⁵ and R⁶ are as in claim 1
is reacted with an iminium salt of the general formula XVI 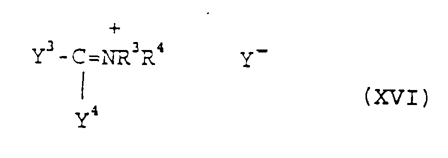 where R³ and R⁴ are as above,
in which Y³ and Y⁴ represent identical or different leaving groups, such as, for example, chlorine, amino groups or alkylmercapto or alkoxy groups, and Y⁻ represents an acid radical anion, for example a halide, a sulphonate, a sulphate or a trifluoroacetate,
and if appropriate with a base;
(11) to prepare compounds of the formula I in which R² denotes R⁷C=Z⁴, wherein R⁷ denotes methyl, methoxy, ethoxy or NH-C₆H₅ and Z⁴ represents an oxygen atom,
a 2-unsubstituted 3-aminopyrrole of the general formula XVII 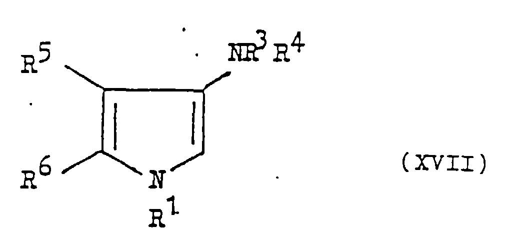 where R¹ and R³ to R⁶ are as in claim 1
is reacted with a carbonyl compound of the general formula XVIII 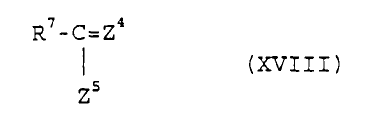 where R⁷ and Z⁴ are as above, in which Z⁵ denotes a leaving group, such as, for example, a halogen or an acyloxy group,
or if, in the compounds of the formula I where R² = R⁷C=Z⁴, R⁷ denotes NHC₆H₅,
also with a heterocumulene of the general formula XIX
R⁵N=C=Z⁴ (XIX)
where Z⁴ and R⁵ are as above
and if appropriate with a Lewis or proton acid;
(12) to prepare compounds of the formula I in which R¹ and R⁶ denote hydrogen and NR³R⁴ denotes morpholino,
a 3-substituted pyrrole of the general formula XX 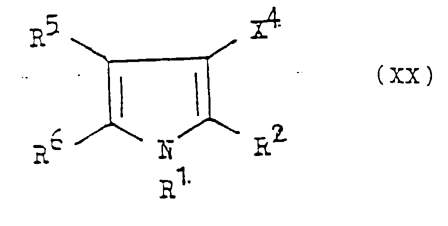 where R¹, R², R⁵ and R⁶ are as in claim 1,
in which X⁴ denotes a leaving group, such as a halogen, a hydroxyl or alkoxy group, an alkylthio group or a diazonium group,
is reacted with an amine of the general formula XXI
HNR³R⁴ (XXI)
where R³ and R⁴ are as in claim 1;
(13) to prepare compounds of the formula I in which R² denotes COOR⁷, wherein R⁷ represents methyl or ethyl,
a 3-aminopyrrolecarboxylic acid of the general formula XXII 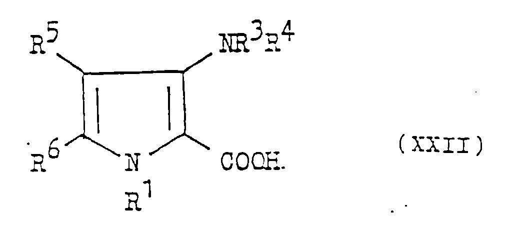 where R¹, R³, R⁴, R⁵ and R⁶ are as in claim 1,
or a salt of a 3-aminopyrrolecarboxylic acid of the general formula XXII, for example a sodium, potassium or ammonium salt, is reacted with an alkylating agent of the general formula XXIII
R⁷X⁵ (XXIII)
where R⁷ is as above, in which X⁵ denotes a leaving group, for example a halogen or a sulphonyl group,
and if appropriate with a base.

2. Process according to claim 1, variants 1 to 9, characterized in that an amine, an alkali metal or alkaline earth metal hydroxide or hydride, an alkali metal carbonate or a metal amide is used as the base.

3. Process according to claim 1, variants 1, 2 and 5, characterized in that an alkyl or aralkyl halide, an alkylsulphonate, a dialkyl sulphate, a trialkyloxonium salt or diazomethane is used as the alkylating agent.

4. Process according to claim 1, variants 1 and 5, characterized in that hydrogen peroxide, a peracid or a persulphate is used as the oxidizing agent.

5. Use of 3-aminopyrroles of the formula I 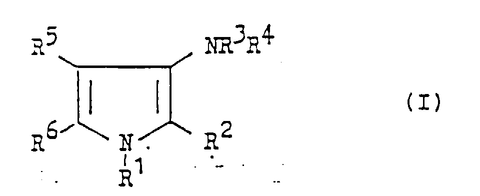 in which the substituents R¹ to R⁶ have the meanings corresponding to the following combinations:
| R¹ | R² | R³ | R⁴ | R⁵ | R⁶ |
|---|---|---|---|---|---|
| H | COOCH₃ | CH₃ | CH₃ | c₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |
as pharmaceutical agents or for the preparation of pharmaceutical agents, in particular having a CNS activity and preferably an anticonvulsive and/or analgesic action.

6. Use of the 3-aminopyrroles of the formula I according to claim 5 for the preparation of pharmaceutical agents for treatment of diseases of the central nervous system, in particular epilepsies in various forms, non-epileptic fits, migraines and pain of various origins.

7. Process for the preparation of pharmaceutical compositions, characterized in that one or more 3-aminopyrroles of the formula I according to claim 5 are brought into a galenically suitable form in a manner known per se using pharmaceutically suitable auxiliaries and/or carriers and/or diluents.

## Revendications (Revendications pour l'(les) Etat(s) contractant(s) suivant(s): BE, CH, LI, DE, FR, GB, IT, NL, SE)

1. 3-aminopyrroles de formule I, 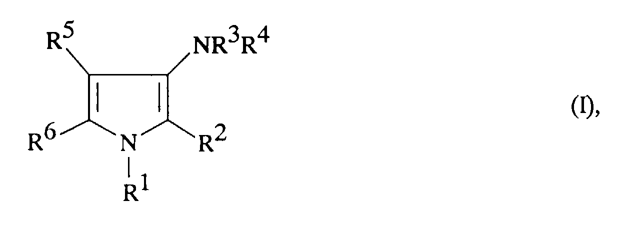 où les substituants R¹ à R⁶ ont les significations correspondant aux combinaisons ci-après :
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |

2. Procédé de préparation des 3-aminopyrroles de formule I selon la revendication 1, caractérisé en ce
(1) qu'un dérivé d'acide aminoacrylique de formule générale II 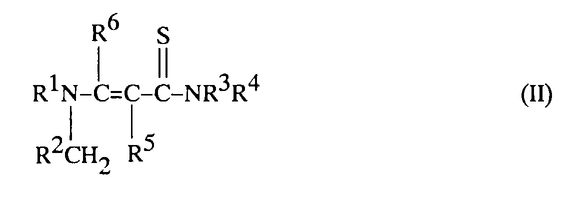 où R¹ à R⁶ sont tels qu'à la revendication 1
est cyclisé,
de préférence en présence d'un agent alkylant, d'un oxydant et de préférence en présence d'une base ;
(2) qu'en vue de la préparation de composés de formule I, où R¹ et R⁶ désignent un atome d'hydrogène et NR³R⁴ un groupe morpholino,
un dérivé d'acide aminoacrylique de formule générale III 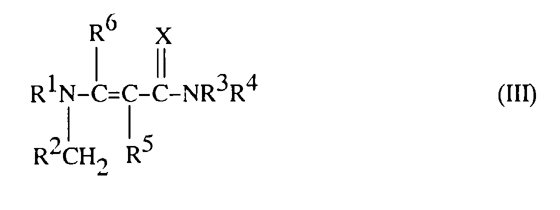 ou R¹ à R⁶ sont tels qu'à la revendication 1,
où X désigne un atome d'oxygène ou un atome d'azote substitué,
est cyclisé, le cas échéant en présence d'un agent alkylant ou d'un déshydratant et de préférence en présence d'une base ;
(3) qu'un sel de triméthinium de formule générale IV 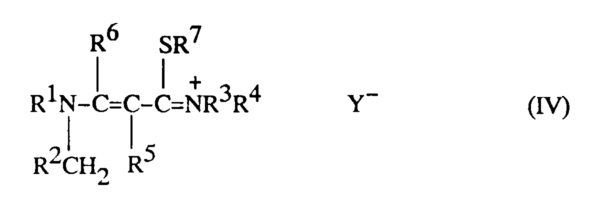 où R¹ à R⁶ sont tels qu'à la revendication 1, où R⁷ est un groupe alkyle ou aralkyle et Y⁻ un anion acide résiduel, par exemple un halogénure, perchlorate, sulfate d'alkyle, sulfonate, sulfate, tétrafluoroborate ou tétraarylborate ou picrate,
ou, le cas échéant, la base libre correspondante d'un sel de triméthinium de formule générale IV
est cyclisé, de préférence avec une base ;
(4) qu'en vue de la préparation de composés de formule I, où R¹ et R⁶ désignent un atome d'hydrogène et NR³R⁴ un groupe morpholino,
un sel de triméthinium de formule générale V 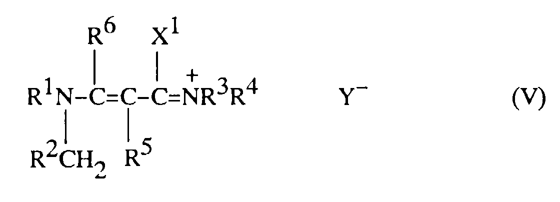 où R¹ à R⁶ sont tels qu'à la revendication 1, où X¹ est un groupe qui part,
par exemple un halogène, un groupe alcoxy ou un groupe amino et Y⁻ est un anion résiduel acide, par exemple un halogénure, perchlorate, sulfate d'alkyle, sulfonate, sulfate, tétrafluoroborate ou tétraarylborate ou picrate, ou, le cas échéant, la base libre correspondante d'un sel de triméthinium de formule générale V
est cyclisé, de préférence en présence d'une base ;
(5) qu'en vue de la préparation de composés de formule I , où R¹ et R⁶ désignent un atome d'hydrogène et NR³R⁴ un groupe morpholino,
un dérivé d'amide d'acide acrylique de formule générale VI 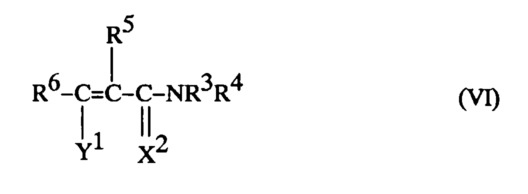 où R³ à R⁶ sont tels qu'à la revendication 1, où X² désigne un atome d'hydrogène, de soufre ou un atome d'azote monosubstitué ou disubstitué et Y¹ un groupe qui part, par exemple un groupe amino substitué ou non substitué, un groupe alkylthio, alcoxy, hydroxyle, mercapto ou acyloxy ou un halogène,
est mis à réagir avec une amine de formule générale VII
R²CH₂NHR¹ (VII)
où R¹ et R² sont tels qu'à la revendication 1,
de préférence avec un agent alkylant ou un oxydant et de préférence avec une base ;
(6) qu'en vue de la préparation de composés de formule I, où R¹ et R⁶ désignent un atome d'hydrogène et NR³R⁴ désigne un groupe morpholino,
un sel d'iminium de formule générale VIII 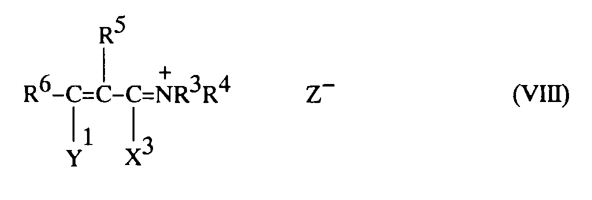 ou R³ à R⁶ sont tels qu'à la revendication 1, où X³ et Y¹ sont identiques ou différents et désignent un groupe qui part, par exemple un groupe amino substitué ou non substitué, un groupe alkylthio, alcoxy, hydroxy, mercapto, acyloxy ou un halogène et Z⁻ un anion résiduel acide, par exemple un halogénure, perchlorate, sulfate d'alkyle, sulfonate, sulfate, tétrafluoroborate ou tétraarylborate ou picrate,
est mis à réagir avec une amine de formule générale VII telle que définie ci-dessus et avec une base ;
(7) qu'en vue de la préparation de composés de formule I, où R¹ désigne un groupe méthyle, éthyle, benzyle ou éthoxycarbonylméthyle,
un 3-aminopyrrole non substitué en 1 de formule générale IX 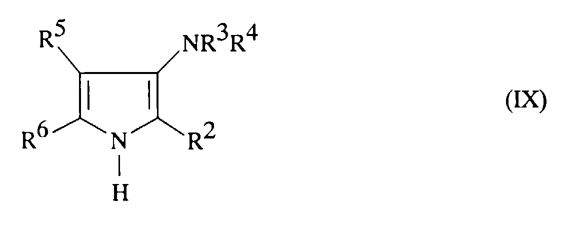 où R² à R⁶ sont tels qu'à la revendication 1,
est mis à réagir avec un agent alkylant de formule générale X
R¹-Y² (X)
où R¹ est tel qu'à la revendication 1, où Y² est un groupe qui part, par exemple un halogène, un groupe sulfonyloxy ou diazo et avec une base ;
(8) qu'en vue de la préparation de composés de formule I, où R² désigne COZ¹ pour Z¹ = un groupe hydroxyle, NaO, méthoxy, éthoxy ou anilino, un dérivé d'acide pyrrole-carboxylique de formule générale XI 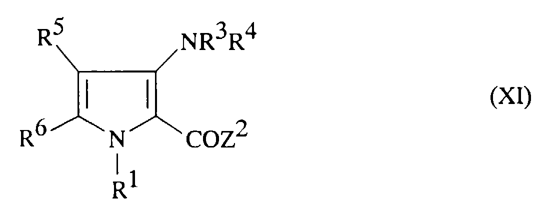 où R¹ à R⁶ sont tels qu'à la revendication 1, où Z², différent de Z¹, désigne un groupe qui part, par exemple un groupe hydroxyle, alcoxy, aryloxy, acyloxy ou alkylthio ou un halogène,
est mis à réagir avec un réactif nucléophile de formule générale XII
Z¹H (XII)
où Z¹ est tel que ci-dessus
ou avec la forme déprotonée d'un réactif nucléophile de formule générale XII et le cas échéant avec un acide ou une base ;
(9) qu'en vue de la préparation de composés de formule I, où R¹ et R⁶ désignent un atome d'hydrogène et NR³R⁴ un groupe morpholino,
une énamine de formule générale XIII 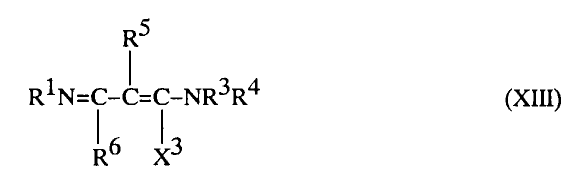 où R¹ à R⁶ sont tels qu'à la revendication 1, où X³ est un groupe qui part, par exemple un groupe alcoxy, alkylmercapto ou amino substitué ou un halogène,
ou un sel d'une énamine de formule générale XIII, par exemple un halogénohydrate, un hydroperchlorate, un hydrosulfonate ou un hydrotétrafluoroborate,
est mis à réagir avec un agent alkylant de formule générale XIV
R²CH₂Z³ (XIV)
où R² est tel que défini ci-dessus, où Z³ désigne un groupe qui part, par exemple un halogène, un sulfonate, un sulfate d'alkyle ou un trifluoroacétate, et avec une base ;
(10) qu'en vue de la préparation de composés de formule I, où R¹ et R⁶ désignent un atome d'hydrogène et NR³R⁴ un groupe morpholino,
une énamine de formule générale XV 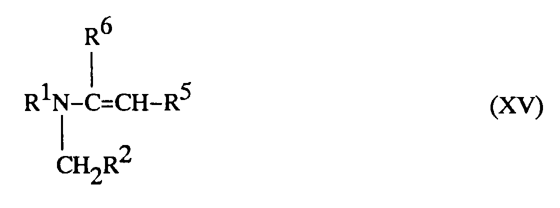 ou R¹, R², R⁵ et R⁶ sont tels que définis à la revendication 1,
est mise à réagir avec un sel d'iminium de formule générale (XVI) 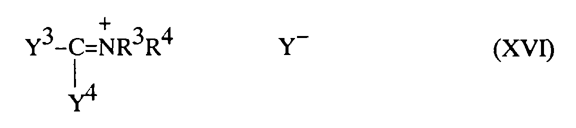 où R³ et R⁴ sont tels que définis ci-dessus, où Y³ et Y⁴ représentent des groupes qui partent identiques ou différents, par exemple un atome de chlore, des groupes amino, alkylmercapto ou alcoxy, et Y⁻ est un anion résiduel acide, par exemple un halogénure, un sulfonate, un sulfate ou un trifluoroacétate,
et le cas échéant avec une base ;
(11) qu'en vue de la préparation de composés de formule I, où R² signifie R⁷C=Z⁴, R⁷ représentant un groupe méthyle, méthoxy, éthoxy ou NH-C₆H₅ et Z⁴ représentant un atome d'oxygène,
un 3-aminopyrrole non substitué en 2 de formule générale XVII 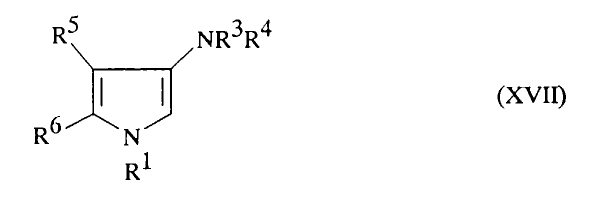 où R¹ et R³ à R⁶ sont tels que définis à la revendication 1,
est mis à réagir avec un composé carbonyle de formule générale XVIII 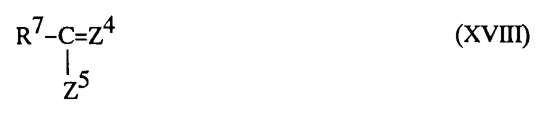 où R⁷ et Z⁴ sont tels que définis ci-dessus, où Z⁵ désigne un groupe qui part, par exemple, un halogène ou un groupe acyloxy,
ou, dans la mesure où dans les composés de formule I où R² = R⁷C=Z⁴, R⁷ représente NH₆H₅,
également avec un hétérocumylène de formule générale XIX
R⁵N=C=Z⁴ (XIX)
où Z⁴ et R⁵ sont tels que définis ci-dessus,
et le cas échéant avec un acide de Lewis ou un acide protonique ;
(12) qu'en vue de la préparation de composés de formule I, où R¹ et R⁶ désignent un atome d'hydrogène et NR³R⁴ un groupe morpholino,
un pyrrole substitué en 3 de formule générale XX 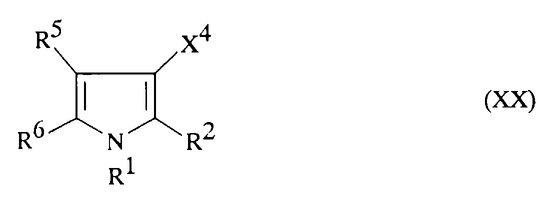 où R¹, R², R⁵ et R⁶ sont tels que définis à la revendication 1, où X⁴ est un groupe qui part, par exemple un halogène, un groupe hydroxy ou alcoxy, un groupe alkylthio ou un groupe diazonium,
est mis à réagir avec une amine de formule générale XXI
HNR³R⁴ (XXI)
où R³ et R⁴ sont tels que définis à la revendication 1 ;
(13) qu'en vue de la préparation de composés de formule I, où R² désigne COOR⁷, R⁷ représentant un groupe méthyle ou éthyle,
un acide 3-aminopyrrole-carboxylique de formule générale XXII 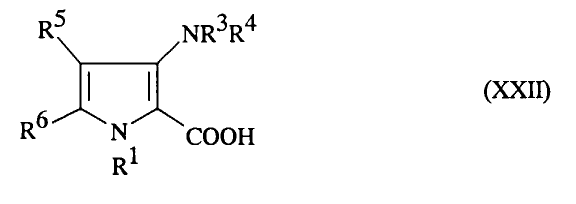 où R¹, R³, R⁴, R⁵ et R⁶ sont tels que définis à la revendication 1,
ou un sel d'un acide 3-aminopyrrole-carboxylique de formule générale XXII, par exemple un sel de sodium, de potassium ou d'ammonium, est mis à réagir avec un agent alkylant de formule générale XXIII
R⁷X⁵ (XXIII)
où R⁷ est tel que défini ci-dessus, où X⁵ désigne un groupe qui part, par exemple un halogène ou un groupe sulfonyle,
et le cas échéant avec une base.

3. Procédé selon la revendication 2, variantes 1 à 9, caractérisé en ce qu'on utilise comme base une amine, un hydroxyde ou un hydrure de métal alcalin ou de métal alcalino-terreux, un carbonate alcalin ou un amide de métal.

4. Procédé selon la revendication 2, variantes 1, 2 et 5, caractérisé en ce qu'on utilise comme agent alkylant un halogénure d'alkyle ou d'aralkyle, un sulfate d'alkyle, un sulfate de dialkyle, un sel de trialkyloxonium ou du diazométhane.

5. Procédé selon la revendication 2, variantes 1 et 5, caractérisé en ce qu'on utilise comme oxydant du peroxyde d'hydrogène, un peracide ou un persulfate.

6. Compositions pharmaceutiques, caractérisées par un ou plusieurs 3-aminopyrroles en tant que principes actifs, outre un ou plusieurs adjuvants et/ou véhicules physiologiquement compatibles et le cas échéant un diluant, les 3-aminopyrroles étant choisis parmi les composés de formule I selon la revendication 1, ainsi que parmi d'autres composés de formule I tels que définis à la revendication 1 ayant les combinaisons suivantes de substituants R¹ à R⁶ :
| R¹ | R² | R³ | R⁴ | R⁵ | R⁶ |
|---|---|---|---|---|---|
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-)CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |

7. Compositions pharmaceutiques selon la revendication 6, ayant notamment une activité neurologique centrale et de préférence un effet anticonvulsivant et/ou analgésique, caractérisées par des aminopyrroles de formule I tels que définis dans la revendication 6 en une quantité totale de 0,5 à 20 mg/kg par dose individuelle.

8. Utilisation des 3-aminopyrroles de formule I selon la revendication 1 ainsi que des autres composés de formule I mentionnés à la revendication 6 comme agents pharmaceutiques destinés au traitement de tableaux cliniques neurologiques centraux, notamment de l'épilepsie sous ses diverses formes, des crises non épileptiques, des migraines et des douleurs d'origine diverses ou pour leur préparation.

9. Procédé de préparation des compositions pharmaceutiques selon la revendication 6 ou 7, caractérisé en ce que le ou les principes actifs sont amenés de façon connue en soi à une forme galéniquement appropriée par utilisation d'adjuvants et/ou de véhicules et/ou de diluants pharmaceutiquement appropriés.

## Revendications (Revendications pour l'(les) Etat(s) contractant(s) suivant(s): ES)

1. Procédé de préparation de 3-aminopyrroles de formule I 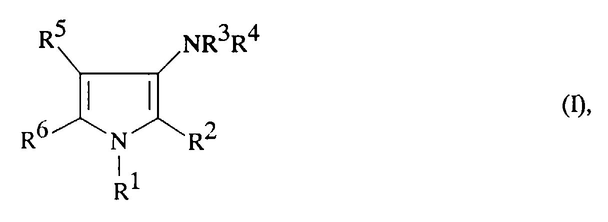 où les substituants R¹ à R⁶ ont les significations correspondant aux combinaisons ci-après :
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-(CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂ )₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
caractérisé en ce
(1) qu'un dérivé d'acide aminoacrylique de formule générale II 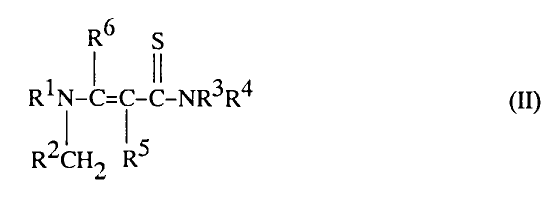 où R¹ à R⁶ sont tels qu'à la revendication 1
est cyclisé.
de préférence en présence d'un agent alkylant, d'un oxydant et de préférence en présence d'une base ;
(2) qu'en vue de la préparation de composés de formule I, où R¹ et R⁶ désignent un atome d'hydrogène et NR³R⁴ un groupe morpholino,
un dérivé d'acide aminoacrylique de formule générale III 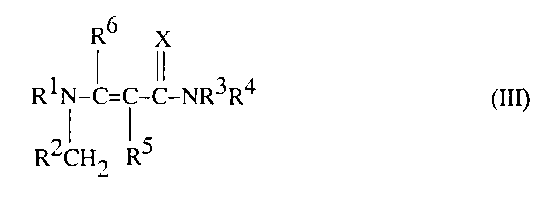 où R¹ à R⁶ sont tels qu'à la revendication 1, où X désigne un atome d'oxygène ou un atome d'azote substitué,
est cyclisé, le cas échéant en présence d'un agent alkylant ou d'un déshydratant et de préférence en présence d'une base ;
(3) qu'un sel de triméthinium de formule générale IV 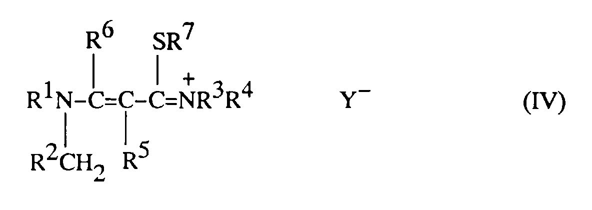 où R¹ à R⁶ sont tels qu'à la revendication 1, où R⁷ est un groupe alkyle ou aralkyle et Y⁻ un anion acide résiduel, par exemple un halogénure, perchlorate, sulfate d'alkyle, sulfonate, sulfate, tétrafluoroborate ou tétraarylborate ou picrate,
ou, le cas échéant, la base libre correspondante d'un sel de triméthinium de formule générale IV
est cyclisé, de préférence avec une base ;
(4) qu'en vue de la préparation de composés de formule I, où R¹ et R⁶ désignent un atome d'hydrogène et NR³R⁴ un groupe morpholino,
un sel de triméthinium de formule générale V 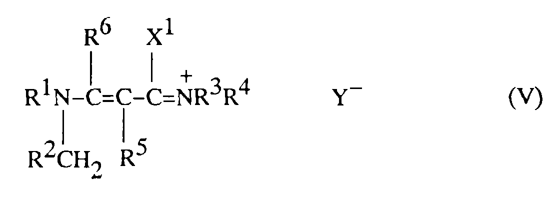 où R¹ à R⁶ sont tels qu'à la revendication 1,où X¹ est un groupe qui part, par exemple un halogène, un groupe alcoxy ou un groupe amino et Y⁻ est un anion résiduel acide, par exemple un halogénure, perchlorate, sulfate d'alkyle, sulfonate, sulfate, tétrafluoroborate ou tétraarylborate ou picrate, ou, le cas échéant la base libre correspondante d'un sel de triméthinium de formule générale V
est cyclisé, de préférence en présence d'une base ;
(5) qu'en vue de la préparation de composés de formule I, où R¹ et R⁶ désignent un atome d'hydrogène et NR³R⁴ un groupe morpholino,
un dérivé d'amide d'acide acrylique de formule générale VI 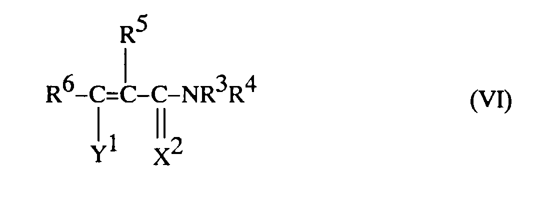 ou R³ à R⁶ sont tels qu'à la revendication 1, où X² désigne un atome d'hydrogène, de soufre ou un atome d'azote monosubstitué ou disubstitué et Y¹ un groupe qui part, par exemple un groupe amino substitué ou non substitué, un groupe alkylthio, alcoxy, hydroxyle, mercapto ou acyloxy ou un halogène,
est mis à réagir avec une amine de formule générale VII
R²CH₂NHR¹ (VII)
où R¹ et R² sont tels qu'à la revendication 1,
de préférence avec un agent alkylant ou un oxydant et de préférence avec une base ;
(6) qu'en vue de la préparation de composés de formule I, où R¹ et R⁶ désignent un atome d'hydrogène et NR³R⁴ désigne un groupe morpholino,
un sel d'iminium de formule générale VIII 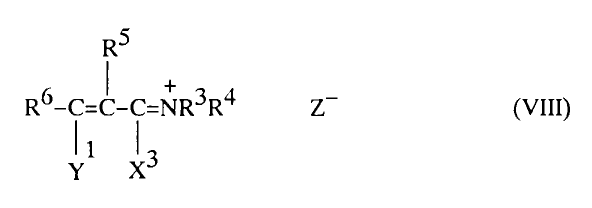 où R³ à R⁶ sont tels qu'à la revendication 1, où X³ et Y¹ sont identiques ou différents et désignent un groupe qui part, par exemple un groupe amino substitué ou non substitué, un groupe alkylthio, alcoxy, hydroxyle, mercapto, acyloxy ou un halogène et Z⁻ un anion résiduel acide, par exemple un halogénure, perchlorate, sulfate d'alkyle, sulfonate, sulfate, tétrafluoroborate ou tétraarylborate ou picrate,
est mis à réagir avec une amine de formule générale VII telle que définie ci-dessus et avec une base ;
(7) qu'en vue de la préparation de composés de formule I, où R¹ désigne un groupe méthyle, éthyle, benzyle ou éthoxycarbonylméthyle,
un 3-aminopyrrole non substitué en 1 de formule générale IX 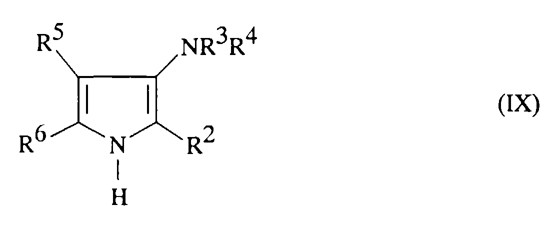 où R² à R⁶ sont tels qu'à la revendication 1,
est mis à réagir avec un agent alkylant de formule générale X
R¹-Y² (X)
où R¹ est tel qu'à la revendication 1, où Y² est un groupe qui part, par exemple un halogène, un groupe sulfonyloxy ou diazo
et avec une base ;
(8) qu'en vue de la préparation de composés de formule I, où R² désigne COZ¹ pour Z¹ = un groupe hydroxyle, NaO, méthoxy, éthoxy ou anilino,
un dérivé d'acide pyrrole-carboxylique de formule générale XI 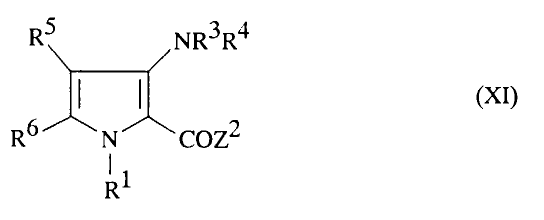 où R¹ à R⁶ sont tels qu'à la revendication 1, où Z², différent de Z¹, désigne un groupe qui part, par exemple un groupe hydroxyle, alcoxy, aryloxy, acyloxy ou alkylthio ou un halogène,
est mis à réagir avec un réactif nucléophile de formule générale XII
Z¹H (XII)
où Z¹ est tel que ci-dessus
ou avec la forme déprotonée d'un réactif nucléophile de formule générale XII et le cas échéant avec un acide ou une base ;
(9) qu'en vue de la préparation de composés de formule I, où R¹ et R⁶ désignent un atome d'hydrogène et NR³R⁴ un groupe morpholino,
une énamine de formule générale XIII 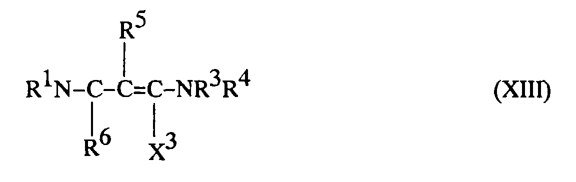 où R¹ à R⁶ sont tels qu'à la revendication 1, où X³ est un groupe qui part, par exemple un groupe alcoxy, mercaptoalkyle ou amine substituée ou un halogène,
ou un sel d'une énamine de formule générale XIII, par exemple un halogénohydrate, un hydroperchlorate, un hydrosulfonate ou un hydrotétrafluoroborate,
est mis à réagir avec un agent alkylant de formule générale XIV
R²CH₂Z³ (XIV)
où R² est tel que défini ci-dessus, où Z³ désigne un groupe qui part, par exemple un halogène, un sulfonate, un sulfate d'alkyle ou un trifluoroacétate, et avec une base ;
(10) qu'en vue de la préparation de composés de formule I, où R¹ et R⁶ désignent un atome d'hydrogène et NR³R⁴ un groupe morpholino, une énamine de formule générale XV 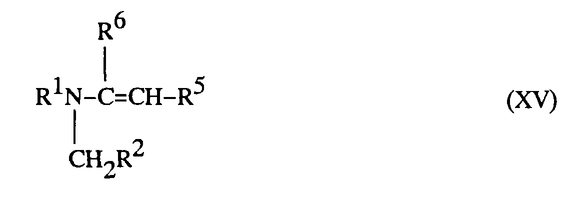 où R¹, R², R⁵ et R⁶ sont tels que définis à la revendication 1,
est mise à réagir avec un sel d'iminium de formule générale (XVI) 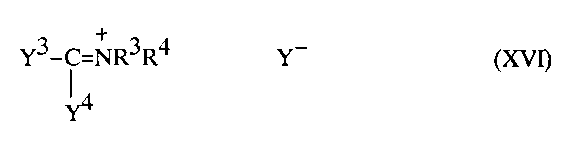 où R³ et R⁴ sont tels que définis ci-dessus, où Y³ et Y⁴ représentent des groupes qui partent identiques ou différents, par exemple un atome de chlore, des groupes amines, alkylmercapto ou alcoxy, et Y⁻ est un anion résiduel acide, par exemple un halogénure, un sulfonate, un sulfate ou un trifluoroacétate,
et le cas échéant avec une base ;
(11) qu'en vue de la préparation de composés de formule I, où R² signifie R⁷C=Z⁴, R⁷ représentant un groupe méthyle, méthoxy, éthoxy ou NH-C₆H₅ et Z⁴ représentant un atome d'oxygène,
un 3-aminopyrrole non substitué en 2 de formule générale XVII 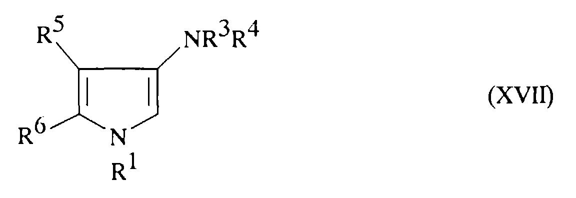 ou R¹ et R³ à R⁶ sont tels que définis à la revendication 1,
est mis à réagir avec un composé carbonyle de formule générale XVIII 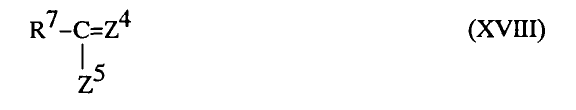 où R⁷ et Z⁴ sont tels que définis ci-dessus, où Z⁵ désigne un groupe qui part, par exemple, un halogène ou un groupe acyloxy,
ou, dans la mesure où dans les composés de formule I où R² = R⁷C=Z⁴, R⁷ représente NH₆H₅,
également avec un hétérocumylène de formule générale XIX
R⁵N=C=Z⁴ (XIX)
où Z⁴ et R⁵ sont tels que définis ci-dessus,
et le cas échéant avec un acide de Lewis ou un acide protonique ;
(12) qu'en vue de la préparation de composés de formule I, où R¹ et R⁶ désignent un atome d'hydrogène et NR³R⁴ un groupe morpholino,
un pyrrole substitué en 3 de formule générale XX 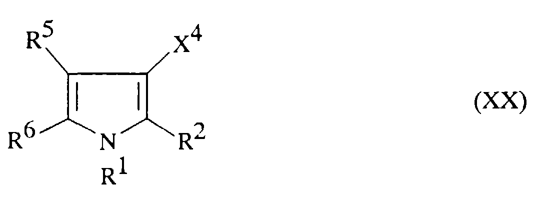 où R¹, R², R⁵ et R⁶ sont tels que définis à la revendication 1, où X⁴ est un groupe qui part, par exemple un halogène, un groupe hydroxyle ou alcoxy, un groupe alkylthio ou un groupe diazonium,
est mis à réagir avec une amine de formule générale XXI
HNR³R⁴ (XXI)
où R³ et R⁴ sont tels que définis à la revendication 1 ;
(13) qu'en vue de la préparation de composés de formule I, où R² désigne COOR⁷, R⁷ représentant un groupe méthyle ou éthyle,
un acide 3-aminopyrrole-carboxylique de formule générale XXII 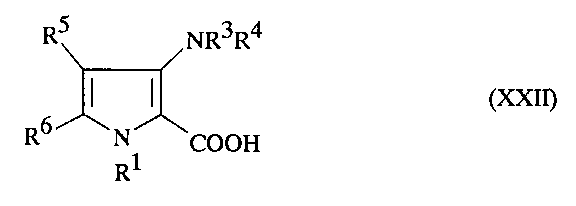 où R¹, R³, R⁴, R⁵ et R⁶ sont tels que définis à la revendication 1,
ou un sel d'un acide 3-aminopyrrole-carboxylique de formule générale XXII, par exemple un sel de sodium, de potassium ou d'ammonium, est mis à réagir avec un agent alkylant de formule générale XXIII
R⁷X⁵ (XXIII)
où R⁷ est tel que défini ci-dessus, où X⁵ désigne un groupe qui part, par exemple un halogène ou un groupe sulfonyle,
et le cas échéant avec une base.

2. Procédé selon la revendication 1, variantes 1 à 9, caractérisé en ce qu'on utilise comme base une amine, un hydroxyde ou un hydrure de métal alcalin ou de métal alcalino-terreux, un carbonate alcalin ou un amide de métal.

3. Procédé selon la revendication 1, variantes 1, 2 et 5, caractérisé en ce qu'on utilise comme agent alkylant un halogénure d'alkyle ou d'aralkyle, un sulfate d'alkyle, un sulfate de dialkyle, un sel de trialkyloxonium ou du diazométhane.

4. Procédé selon la revendication 1, variantes 1 et 5, caractérisé en ce qu'on utilise comme oxydant du peroxyde d'hydrogène, un peracide ou un persulfate.

5. Utilisation de 3-aminopyrroles de formule I 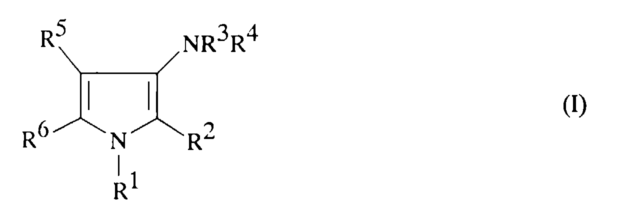 où les substituants R¹ à R⁶ ont les significations correspondant aux combinaisons ci-après :
| R¹ | R² | R³ | R⁴ | R⁵ | R⁶ |
|---|---|---|---|---|---|
| H | COOCH₃ | CH₃ | CH₃ | C₆H₅ | H |
| H | COOC₂H₅ | H | C₆H₅ | (CH₂)₄ | |
| H | COOCH₃ | (CH₂)₄ | | C₆H₅ | H |
| H | COOC₂H₅ | (CH₂)₄ | | C₆H₅ | H |
| H | COOCH₃ | (CH₂)₅ | | 4-ClC₆H₄ | H |
| H | COOC₂H₅ | (CH₂)₅ | | 4-CH₃C₆H₄ | H |
| H | COOH | (CH₂)₂-O-(CH₂)₂ | | C₆H₅ | H |
| H | COONa | (CH₂)₂-O-(CH₂)₂ | | C₆H₅ | H |
| H | COOCH₃ | (CH₂)₂-O-(CH₂)₂ | | 3-BrC₆H₄ | H |
| H | COOCH₃ | (CH₂)₂-O-( CH₂)₂ | | 4-BrC₆H₄ | H |
| H | COOCH₃ | (CH₂)₂-O-(CH₂)₂ | | 4-CH₃CH₂C₆H₄ | H |
| H | COOCH₃ | (CH₂)₂-O-(CH₂)₂ | | 4-FC₆H₄ | H |
| H | COOCH₃ | (CH₂)₂-O-(CH₂)₂ | | 3-CH₃OC₆H₄ | H |
| H | COOCH₃ | (CH₂)₂-O-(CH₂)₂ | | 4-C₆H₅C₆H₄ | H |
| H | COOCH₃ | (CH₂)₂-O-(CH₂)₂ | | 3,4-(CH₃O)₂C₆H₃ | H |
| H | COOCH₂C₆H₅ | (CH₂)₂-O-(CH₂)₂ | | C₆H₅ | H |
| H | CONHC₆H₅ | (CH₂)₂-O-(CH₂)₂ | | C₆H₅ | H |
| H | COCH₃ | (CH₂)₂-O-(CH₂)₂ | | 4-ClC₆H₄ | H |
| CH₃ | COOCH₃ | (CH₂)₂-O-(CH₂)₂ | | C₆H₅ | H |
| CH₃ | COOCH₃ | (CH₂)₂-O-(CH₂)₂ | | 4-CH₃C₆H₄ | H |
| CH₃ | COOCH₃ | (CH₂)₂-O-(CH₂)₂ | | 4-ClC₆H₄ | H |
| CH₃ | COOC₂H₅ | (CH₂)₂-O-(CH₂)₂ | | 4-ClC₆H₄ | H |
| CH₃ | COOCH₃ | (CH₂)₂-O-(CH₂)₂ | | 4-BrC₆H₄ | H |
| C₂H₅ | COOCH₃ | (CH₂)₂-O-(CH₂)₂ | | 4-BrC₆H₄ | H |
| CH₂C₆H₅ | COOCH₃ | (CH₂)₂-O-(CH₂)₂ | | C₆H₅ | H |
| CH₂C₆H₅ | COOCH₃ | (CH₂)₂-O-(CH₂)₂ | | 4-ClC₆H₄ | H |
| CH₂COOC₂H₅ | COOCH₃ | (CH₂)₂-O-(CH₂)₂ | | C₆H₅ | H |
| H | COOCH₃ | (CH₂)₂-O-(CH₂)₂ | | C₆H₅ | H |
| H | COOC₂H₅ | (CH₂)₂-O-(CH₂)₂ | | C₆H₅ | H |
| H | COOCH₃ | (CH₂)₂-O-(CH₂)₂ | | 4-CH₃C₆H₄ | H |
| H | COOC₂H₅ | (CH₂)₂-O-(CH₂)₂ | | 4-CH₃C₆H₄ | H |
| H | COOCH₃ | (CH₂)₂-O-(CH₂)₂ | | 4-CH₃OC₆H₄ | H |
| H | COOC₂H₅ | (CH₂)₂-O-(CH₂)₂ | | 4-CH₃OC₆H₄ | H |
| H | COOCH₃ | (CH₂)₂-O-(CH₂)₂ | | 4-ClC₆H₄ | H |
| H | COOC₂H₅ | (CH₂)₂-O-(CH₂)₂ | | 4-ClC₆H₄ | H |
comme agents pharmaceutiques ou pour la préparation d'agents pharmaceutiques, ayant notamment une activité neurologique centrale ou de préférence un effet anticonvulsivant et/ou analgésique.

6. Utilisation de 3-aminopyrroles de formule I selon la revendication 5, pour la préparation d'agents pharmaceutiques destinés au traitement de tableaux cliniques neurologiques centraux, notamment de l'épilepsie sous ses diverses formes, des crises non épileptiques, des migraines et des douleurs d'origines diverses.

7. Procédé de préparation de compositions pharmaceutiques, caractérisé en ce qu'un ou plusieurs 3-aminopyrroles de formule I selon la revendication 5 sont amenés de façon connue en soi à une forme galéniquement appropriée par utilisation d'adjuvants et/ou de véhicules et/ou de diluants pharmaceutiquement appropriés.
